(19) **Europäisches Patentamt**
**European Patent Office**
**Office européen des brevets**

(11) **EP 2 174 660 B1**

(12) **EUROPEAN PATENT SPECIFICATION**

(45) Date of publication and mention
of the grant of the patent:
**12.12.2012 Bulletin 2012/50**

(21) Application number: **08790688.9**

(22) Date of filing: **27.06.2008**

(51) Int Cl.:
*A61K 38/22* (2006.01)   *A61K 47/42* (2006.01)
*A61P 43/00* (2006.01)

(86) International application number:
**PCT/JP2008/061723**

(87) International publication number:
**WO 2009/005000 (08.01.2009 Gazette 2009/02)**

(54) **COMPOSITION FOR MUCOSAL ADMINISTRATION CONTAINING AGENT FOR ENHANCING MUCOSAL ABSORPTION OF PEPTIDE DRUG AND ADMINISTRATION METHOD THEREOF**

ZUSAMMENSETZUNG ZUR MUKOSALEN VERABREICHUNG MIT EINEM WIRKSTOFF ZUR ERHÖHUNG DER SCHLEIMHAUTABSORPTION EINER PEPTIDARZNEI UND VERFAHREN ZU IHRER VERABREICHUNG

COMPOSITION POUR ADMINISTRATION MUCOSALE CONTENANT UN AGENT D'AMPLIFICATION DE L'ABSORPTION MUCOSALE DE MÉDICAMENT PEPTIDIQUE ET SON PROCÉDÉ D'ADMINISTRATION

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **29.06.2007 JP 2007171676**

(43) Date of publication of application:
**14.04.2010 Bulletin 2010/15**

(73) Proprietor: **Daiichi Sankyo Company, Limited**
**Chuo-ku**
**Tokyo (JP)**

(72) Inventors:
• **KONDOH, Masuo**
**Ikeda-shi**
**Osaka 563-0029 (JP)**
• **UCHIDA, Hiroshi**
**Ohra-gun**
**Gunma 370-0503 (JP)**
• **HANADA, Takeshi**
**Ohra-gun**
**Gunma 370-0503 (JP)**

• **HOSHINO, Masato**
**Ohra-gun**
**Gunma 370-0503 (JP)**

(74) Representative: **Hiebl, Inge Elisabeth et al**
**Kraus & Weisert**
**Patent- und Rechtsanwälte**
**Thomas-Wimmer-Ring 15**
**80539 München (DE)**

(56) References cited:
**WO-A2-2006/047214     JP-A- 06 107 557**
**US-A- 5 695 956     US-A1- 2006 084 594**

• **KONDOH M. ET AL.: 'A NOVEL STRATEGY FOR THE ENHANCEMENT OF DRUG ABSORPTION USING A CLAUDIN MODULATOR' MOL. PHARMACOL. vol. 67, 2005, pages 749 - 756, XP008128160**
• **VAN ITALLIE C.M. ET AL.: 'STRUCTURE OF THE CLAUDIN-BINDING DOMAIN OF CLOSTRIDIUM PERFRIGENS ENTEROTOXIN' J. BIOL. CHEM. vol. 283, January 2008, pages 268 - 274, XP008128574**

SEQUENCE LISTING

<110> ASUBIO PHARMA CO., LTD.

<120> Composition for Mucosal Administration Containing Agent for Accelerating Mucosal Absorption of Peptide Drug and Administration Method Thereof

<130> 20758EP

<140> EP 08 790 688.9
<141> 2008-06-27

<150> JP 2007-171676
<151> 2007-06-29

<160> 4

<170> PatentIn version 3.1

<210> 1
<211> 136
<212> PRT
<213> Clostridium perfringenes NCTC8239 strain

<220>
<221> PEPTIDE
<222> (1)..(136)
<223> Amino acid sequence obtained from Clostridium perfringenes NCTC8239 strain

<400> 1

```
Glu Arg Cys Val Leu Thr Val Pro Ser Thr Asp Ile Glu Lys Glu Ile
1               5                   10                  15

Leu Asp Leu Ala Ala Ala Thr Glu Arg Leu Asn Leu Thr Asp Ala Leu
            20                  25                  30

Asn Ser Asn Pro Ala Gly Asn Leu Tyr Asp Trp Arg Ser Ser Asn Ser
            35                  40                  45

Tyr Pro Trp Thr Gln Lys Leu Asn Leu His Leu Thr Ile Thr Ala Thr
    50                  55                  60

Gly Gln Lys Tyr Arg Ile Leu Ala Ser Lys Ile Val Asp Phe Asn Ile
65                  70                  75                  80

Tyr Ser Asn Asn Phe Asn Asn Leu Val Lys Leu Glu Gln Ser Leu Gly
                85                  90                  95

Asp Gly Val Lys Asp His Tyr Val Asp Ile Ser Leu Asp Ala Gly Gln
            100                 105                 110

Tyr Val Leu Val Met Lys Ala Asn Ser Ser Tyr Ser Gly Asn Tyr Pro
        115                 120                 125

Tyr Ser Ile Leu Phe Gln Lys Phe
    130                 135
```

<210> 2
<211> 411
<212> PRT
<213> Clostridium perfringenes NCTC8239 strain

<220>
<221> DNA
<222> (1)..(411)
<223> DNA obtained from Clostridium perfringenes NCTC8239 strain

<400> 2

```
gagagatgtg ttttaacagt tccatctaca gatatagaaa aagaaatcct tgatttagct    60
gctgctacag aaagattaaa tttaactgat gcattaaact caaatccagc tggtaattta   120
tatgattggc gttcttctaa ctcataccct tggactcaaa agcttaattt acacttaaca   180
attacagcta ctggacaaaa atatagaatc ttagctagca aaattgttga ttttaatatt   240
tattcaaata attttaataa tctagtgaaa ttagaacagt ccttaggtga tggagtaaaa   300
gatcattatg ttgatataag cttagatgct ggacaatatg ttcttgtaat gaaagctaat   360
tcatcatata gtggaaatta cccttattca atattatttc aaaaatttta a           411
```

<210> 3
<211> 126
<212> PRT
<213> Clostridium perfringenes NCTC8239 strain

<220>
<221> PEPTIDE
<222> (1)..(126)
<223> Amino acid sequence obtained from Clostridium perfringenes NCTC8239
strain

<400> 3

```
Asp Ile Glu Lys Glu Ile Leu Asp Leu Ala Ala Ala Thr Glu Arg Leu
1               5                   10                  15

Asn Leu Thr Asp Ala Leu Asn Ser Asn Pro Ala Gly Asn Leu Tyr Asp
            20                  25                  30

Trp Arg Ser Ser Asn Ser Tyr Pro Trp Thr Gln Lys Leu Asn Leu His
        35                  40                  45

Leu Thr Ile Thr Ala Thr Gly Gln Lys Tyr Arg Ile Leu Ala Ser Lys
        50                  55                  60

Ile Val Asp Phe Asn Ile Tyr Ser Asn Asn Phe Asn Asn Leu Val Lys
65                  70                  75                  80

Leu Glu Gln Ser Leu Gly Asp Gly Val Lys Asp His Tyr Val Asp Ile
                85                  90                  95

Ser Leu Asp Ala Gly Gln Tyr Val Leu Val Met Lys Ala Asn Ser Ser
            100                 105                 110

Tyr Ser Gly Asn Tyr Pro Tyr Ser Ile Leu Phe Gln Lys Phe
        115                 120                 125
```

<210> 4
<211> 115
<212> PRT
<213> Clostridium perfringenes NCTC8239 strain

<220>
<221> PEPTIDE
<222> (1)..(115)
<223> Amino acid sequence obtained from Clostridium perfringenes NCTC8239 strain

<400> 4

```
Ala Thr Glu Arg Leu Asn Leu Thr Asp Ala Leu Asn Ser Asn Pro Ala
1               5               10              15

Gly Asn Leu Tyr Asp Trp Arg Ser Ser Asn Ser Tyr Pro Trp Thr Gln
            20              25              30

Lys Leu Asn Leu His Leu Thr Ile Thr Ala Thr Gly Gln Lys Tyr Arg
        35              40              45

Ile Leu Ala Ser Lys Ile Val Asp Phe Asn Ile Tyr Ser Asn Asn Phe
    50              55              60

Asn Asn Leu Val Lys Leu Glu Gln Ser Leu Gly Asp Gly Val Lys Asp
65              70              75              80

His Tyr Val Asp Ile Ser Leu Asp Ala Gly Gln Tyr Val Leu Val Met
            85              90              95

Lys Ala Asn Ser Ser Tyr Ser Gly Asn Tyr Pro Tyr Ser Ile Leu Phe
        100             105             110

Gln Lys Phe
        115
```

**Description**

TECHNICAL FIELD

The present invention relates to a substance having an amino acid sequence of the C-terminal fragment (C-CPE) of an enterotoxin (CPE) produced by *Clostridium perfringens,* a bacterium belonging to the genus Clostridium for use in a method for enhancing mucosal absorption of a peptide drug. In particular, the substance for use in a method for enhancing mucosal absorption of a peptide drug uses C-CPE or a mutant of C-CPE resulting from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE.

BACKGROUND ART

Unlike drugs based on low-molecular-weight compounds, physiologically active peptides, such as insulin, parathyroid hormone and glucagon-like peptide GLP-1, have a molecular weight of several kilodaltons (kDa) and are polar molecules and therefore cannot pass through the epithelium of intestinal mucosa, nasal mucosa, respiratory tract mucosa and cell layers of other mucous membranes. In addition, these peptides are quickly digested by proteases secreted by the mucous epithelial cells. Since these peptide drugs are not absorbed when administered orally, their clinical administration routes are limited to injections such as intramuscular, subcutaneous or intravenous injections. However, the administration by injection is an invasive technique that causes significant pain and mental stress in patients, especially during long-term, repetitive administration. Furthermore, since many of the applications of physiologically active peptides are intended to improve the QOL of patients and thus require long-term, continuous administration of the drugs, there is a need for preparations provided in a dosage form that can be administered at home by patients.

Attempts have been made thus far to use absorption-enhancing agents to improve absorption of high-molecular-weight drugs, or to temporarily increase the permeability of peptide drugs through the mucosa of the digestive tract and other absorption sites. Although different absorption-enhancing agents are known, including fatty acids, such as capric acid and oleic acid, bile acid and chelating agents, such as EDTA, all of them show strong toxicity against mucosal epithelial cells and are not suitable for long-term administration. In addition, the ability of these absorption-enhancing agents to enhance the absorption of peptide drugs can vary significantly depending on the type of absorption site and the physical properties of a particular absorption-enhancing agent.

Thus, in an attempt to improve the mucosal permeation of peptide drugs, we have employed a cell biological approach to develop a substance for the above use that can not only enhance biological absorption of peptide drugs, but also exhibit little or no toxicity against mucosal epithelial tissue as seen with conventional absorption-enhancing agents and are therefore suitable for long-term or repetitive administration.

Neighboring cells in the mucosal epithelial cell layer are joined to each other via an adhesive apparatus called a tight junction, which forms a barrier tight enough to separate even ion molecules. One cell biological approach that has been attempted is to control the function of tight junctions to enhance the absorption of drugs. For example, the extracellular loop peptide of occludin, one of the major constituent proteins of a tight junction, can be used to increase the permeability to dextran up to 40 kDa (Non-Patent Document 1).

Nonetheless, the report is based on the results of an experiment performed on the renal epithelial cells of *Xenopus laevis*: enhancement of the permeability was not confirmed in the experiments using Caco-2 cultured cell layer that reflect the functions of the mucosal epithelium of the human intestinal tract (Non-Patent Documents 2 and 3). Thus, although occludin may cause opening of tight junctions, no evidence exists suggesting the enhancement of biological absorption by occludin.

Claudin, another major membrane protein to form a tight junction, has been identified. So far, 24 members of the human claudin family have been reported.

Unlike occludin, the types and expression patterns of claudins can vary from tissue to tissue. It is known that high levels of claudin-4 are expressed in the epithelium of mucous membranes, including intestinal mucosa and nasal mucosa.

Thus, claudins, as opposed to occludin, are considered to be a potential target for a tissue-specific drug delivery system

A study conducted by Sonoda et al. in 1999 has revealed that an enterotoxin (CPE) produced by *Clostridium perfringens,* one of the food-poisoning bacteria also known as Clostridium welchii, acts specifically on claudin-4 to open tight junctions (Non-Patent Document 4).

It has also been shown that the binding of CPE to claudin-4 involves only the C-terminal fragment of CPE (C-CPE). CPE is also found to act on claudins-3, 6, 7, 8 and 14 of the claudin family, though its binding activity is weaker than to claudin-4.

Kondoh et al. studied whether C-CPE has the ability to enhance mucosal absorption using dextran having molecular weights of 4 kDa, 10 kDa, 20 kDa and 40 kDa and reported that C-CPE enhances the mucosal absorption of dextran if the molecules are 10 kDa or smaller in size (Non-Patent Document 5). Dextran is a polymer of known molecular size and has been used as a marker to study the permeability of tight junctions because of its simple molecular structure and high stability that makes it less susceptible to digestion by proteases and other enzymes present in the mucosa and blood. The 10 kDa dextran used in the experiment had a molecular size of 2.3 nm, which is the estimated size of the opening in a tight junction. Non-Patent Document 5 also mentions that capric acid, a conventional absorption-enhancing agent already in clinical use, exhibits cytotoxicity and causes tissue damage both in the Caco-2 cell line model and in the rat intestinal tract tissue, whereas C-CPE causes little cytotoxicity and tissue damage even at a dose that gives similarly high activity to enhance mucosal absorption, indicating high safety of C-CPE.

However, the experiment described in Non-Patent Document 5 merely uses dextran, which is a polymer and less susceptible to digestion in the mucosa and blood, to determine the molecular size and weight of a substance whose permeation through the opening of tight junctions can be enhanced by C-CPE. Such an experiment does not prove that a given substance having a similar molecular size and weight permeates through tight junctions at different sites in a living body. Once administered to a living body, drugs are readily digested at different sites in the living body by proteases, which are especially abundant in the mucosal epithelial tissue. Peptide drugs are particularly susceptible to digestion by enzymes and their mucosal absorption is largely dependent on their stability in a living body. In addition, because of their complex three-dimensional structure, peptide drugs that have a similar molecular size and weight to the dextrin molecule whose permeation was confirmed in Non-Patent Document 5 may or may not permeate the mucosa. Even if permeable to the mucosa, peptide drugs will be subjected to digestion before and after the permeation. Thus, there is no knowing whether a given peptide drug can be absorbed by a living body.

Non-Patent Document 1: Wong V., Gumbiner BM., Journal of Cell Biology, Vol. 136 pp. 399-409 (1997)
Non-Patent Document 2: Tavelin S., et al., Molecular Pharmacology, Vol. 64 pp. 1530-1540 (2003)
Non-Patent Document 3: Kondoh M., et al., Yakugaku Zasshi Vol.127, No. 4 pp. 601-609 (2007)
Non-Patent Document 4: Journal of Cell Biology, Vol.147, No. 1 pp. 195-204 (1999)
Non-Patent Document 5: Kondoh M., et al., Molecular Pharmacology, Vol. 67 pp. 749-756, (2005)
Non-Patent Document 6: Van Itallie CM., et al., Journal of Biological Chemistry, Vol. 283, pp. 268-274
Non-Patent Document 7: Masuyama A., et al, Journal of Pharmacology and Experimental Therapeutics, 314, pp. 789-95 (2005)
Non-Patent Document 8: Ebihara C., et al, Biochemical Pharmacology, 73, 824-30 (2007)
Non-Patent Document 9: Harada M., et al, Biochemical Pharmacology, 73, pp. 206-14 (2007)
Non-Patent Document 10: Takahashi A., et al, Biochemical Pharmacology, 75, pp. 1639-48 (2008)
Patent Document D1: US 5 695 956 A (McClane, Bruce A [US] et al.), 1997-12-09.
Patent Document D2: US 2006/084594 A1 (Santin, Alessandro D [US] et al.), 2006-04-20.
Patent Document D3: WO 2006/047214 A2 (IGF Oncology LLC [US]; McTavish, Hugh [US]), 2006-05-04.
Patent Document D5 (European family member: D5a):

D5: JP 06 107557 A
D5a: EP 0 474 453

The present inventors investigated the peptide drugs, which are readily digested by proteases abundant in the mucosal epithelial tissue, are unstable, and have a complex three-dimensional structure, by selecting experimental models, varying the concentrations of C-CPE or a C-CPE mutant and peptide drugs to be administered and the timing of administration, and the establishing techniques for detecting absorbed peptide drugs. As a result, the present inventors have found that C-CPE, in particular, C-CPE or mutants of C-CPE resulting from the substitution and/or deletion of one or several amino acid residues of C-CPE, can enhance mucosal permeation of the peptide drugs when co-administered with the peptide drugs at a desirable concentration. The finding ultimately led to the present invention that offers a substance for use in a method for effectively enhancing the mucosal absorption of peptide drugs.

DISCLOSURE OF THE INVENTION

PROBLEMS TO BE SOLVED BY THE INVENTION

Accordingly, it is an objective of the present invention to find, by a cell biological approach, a substance for use in a method that can significantly improve the biological absorption of peptide drugs, which are otherwise substantially unabsorbable by the mucosal epithelium, and that do not cause damage to the mucosal epithelium. It is another object of the present invention to provide a composition for mucosal administration that enhances the mucosal absorption of peptide drugs administered through oral, nasal or pulmonary routes.

MEANS FOR SOLVING THE PROBLEMS

To achieve the above-described objects, one essential aspect of the present invention provides a substance having an amino acid sequence of a C-terminal fragment (C-CPE) of an enterotoxin (CPE) produced by the bacterium *Clostridium perfringens* of the genus Clostridium for use in a method for enhancing mucosal absorption of a peptide drug. Specifically, the present invention concerns the substance for use in a method for enhancing mucosal absorption of a peptide drug, in which the substance having the amino acid sequence of the C-terminal fragment (C-CPE) of the enterotoxin (CPE) is C-CPE or a mutant resulting from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE.

More specifically, the present invention concerns the mucosal absorption-enhancing agent, in which an amino acid sequence of the C-CPE is a sequence represented by SEQ ID NO: 1. The present invention also concerns the substance for use in the above method, in which a base sequence of the C-CPE is a sequence represented by SEQ ID NO: 2.

The present invention concerns the substance for use in a method for enhancing mucosal absorption of a peptide drug, in which the deletion mutant of the C-CPE is a mutant resulting from deletion of one or several amino acid residues from amino acid residues 1 to 21 of the N-terminal of the C-CPE.

The present invention further concerns the substance for use in a method for enhancing mucosal absorption of a peptide drug, in which the deletion mutant of the C-CPE is a mutant resulting from deletion of amino acid residues 1 to 10 of the N-terminal of the C-CPE or a mutant resulting from deletion of amino acid residues 1 to 21 of the N-terminal of the C-CPE.

More specifically, the present invention concerns the substance for use in a method for enhancing mucosal absorption of a peptide drug, in which an amino acid sequence of the mutant of the C-CPE is a sequence represented by SEQ ID NO: 3 or SEQ ID NO: 4.

Another aspect of the present invention provides a composition for mucosal absorption of a peptide drug, using the above-described substance for use in the above-described method. Specifically, the present invention concerns the composition for mucosal administration, containing a peptide drug and the above-described substance as mucosal absorption-enhancing agent.

More specifically, the present invention concerns the composition for mucosal administration, in which the peptide drug is preferably a peptide hormone.

More specifically, the present invention concerns the composition for mucosal administration, in which the peptide hormone is any of parathyroid hormone (PTH) and derivatives thereof, glucagon-like peptide-1, ghrelin, atrial natriuretic peptide, brain natriuretic peptide (BNP), C-type natriuretic peptide, insulin, motilin, leptin, resistin, glucagon, relaxin, galanin, gastrin, apelin, selectin, calcitonin, adrenomedullin, amylin, humanin, thymosin, endorphin, endomorphin, nocistatin, enkephalin, neuropeptide Y, neuropeptide S, neuromedin U, angiotensin, endothelin, guanylin, salusin, urotensin, oxytocin, vasopressin, neurophysin, melanocyte-stimulating hormone, urocortin, lipotropin, luteinizing hormone-releasing hormone, mystatin, prolactin-releasing peptide, somatostatin, cortistatin, thyrotropin-releasing hormone, substance P, neurokinin, endokinin, neurotensin, neoromedin N, obestatin, orexin, insulin-like growth factor-1 (IGF-1), melanin-concentrating hormone, corticotropin-releasing hormone, exendin-4, catacalcin, cholecystokinin, corticotrophin, melanotrophin, neoromedin C, copeptin, pituitary adenylate cyclase-activating peptide (PACAP), peptide YY, thyroliberin and derivatives thereof. More specifically, the present invention concerns the composition for mucosal administration, in which the peptide hormone is human parathyroid hormone or a derivative thereof (hPTH (1-34)), human ghrelin or human motilin.

The present invention further concerns the composition for mucosal administration of a peptide drug, in which the mucosal administration is via intestinal epithelial mucosa, nasal epithelial mucosa, respiratory tract epithelial mucosa or alveolar epithelial mucosa. The present invention further concerns the composition for mucosal administration, provided in the form of a powder preparation, an aqueous suspension or an oil suspension.

Another aspect of the present invention provides a substance for use in a method for enhancing the biological absorption of a peptide drug using the above-described mucosal absorption-enhancing agent. More specifically, the present invention concerns a substance for use in a method for enhancing the biological absorption of a peptide drug through mucosa, comprising co-administering the mucosal absorption-enhancing agent and the peptide drug or separately administering them at an interval. Still more specifically, the present invention concerns a substance for use in a method for enhancing the biological absorption of a peptide drug through mucosa, comprising administering the mucosal absorption-enhancing agent before the peptide drug is administered. Preferably, the present invention concerns a substance for use in a method for enhancing the biological absorption of a peptide drug through mucosa, comprising administering the mucosal absorption-enhancing agent at least two hours before the peptide drug is administered.

The present invention also concerns the method for enhancing the biological absorption of a peptide drug through mucosa, in which the mucosal absorption-enhancing agent is administered at least four hours before the peptide drug is administered.

Most specifically, the present invention concerns the method for enhancing the biological absorption of a peptide drug, in which the mucosal absorption occurs via intestinal epithelial mucosa, nasal epithelial mucosa, respiratory tract epithelial mucosa or alveolar epithelial mucosa. The present invention concerns the method for enhancing the biological absorption of a peptide drug, in which the peptide drug is human parathyroid hormone or a derivative thereof (hPTH (1-34)), human ghrelin or human motilin.

EFFECTS OF THE INVENTION

According to the present invention, the mucosal absorption of peptide drugs via intestinal, pulmonary or nasal route can be enhanced by allowing both the peptide drugs and the C-terminal fragment (C-CPE) of an enterotoxin (CPE) produced by the bacterium *Clostridium perfringens* of the genus Clostridium, in particular, both the peptide drugs and the C-CPE or mutants of C-CPE resulting from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE, to act thereon. The absorption of the peptide drugs can be further improved by administering the C-CPE or its mutants prior to the administration of the peptide drugs. The composition for mucosal administration of the present invention containing the C-CPE or its mutants can be administered via oral, nasal, pulmonary or other administration routes that are less stressful to patients.

More specifically, the present invention enables non-invasive mucosal administration of peptide drugs containing physiologically active, natural or non-natural amino acids, as well as of chemically modified peptide drugs, with high bioavailability.

The composition for mucosal administration of the present invention achieves significantly improved absorption of peptide drugs, such as hPTH(1-34), human ghrelin and human motilin, through the mucosa of small intestine, lung, nasal cavity and other mucosa. Unlike any of the conventional mucosal absorption-enhancers, the composition for mucosal administration of the present invention enables highly effective absorption of peptide drugs by a living body as it does not cause tissue damage and is thus highly safe for use.

BRIEF DESCRIPTION OF DRAWINGS

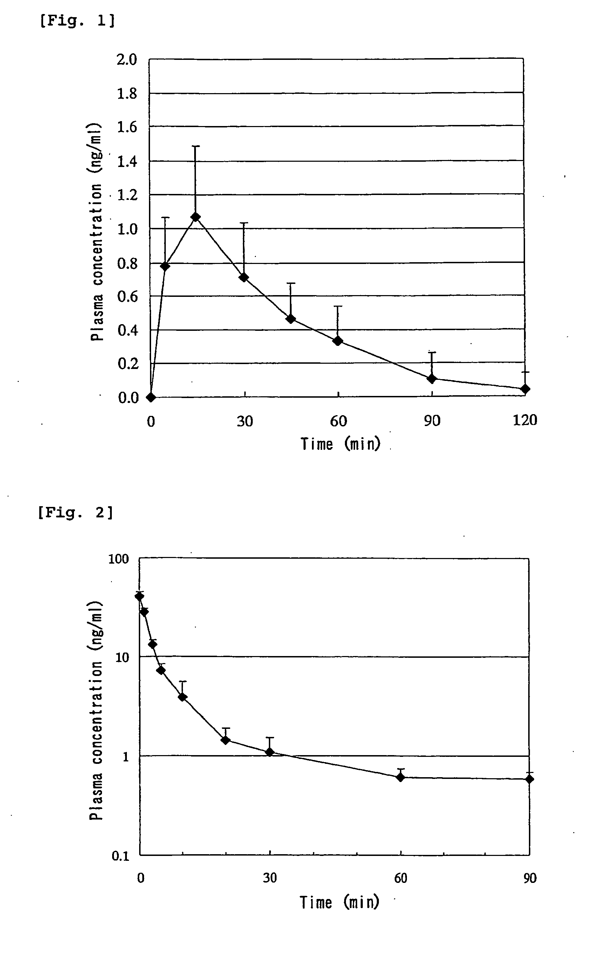

Fig. 1 is a graph showing changes in the plasma concentration after subcutaneous administration of hPTH(1-34) to rats.

Fig. 2 is a graph showing changes in the plasma concentration after intravenous administration of human ghrelin to rats.

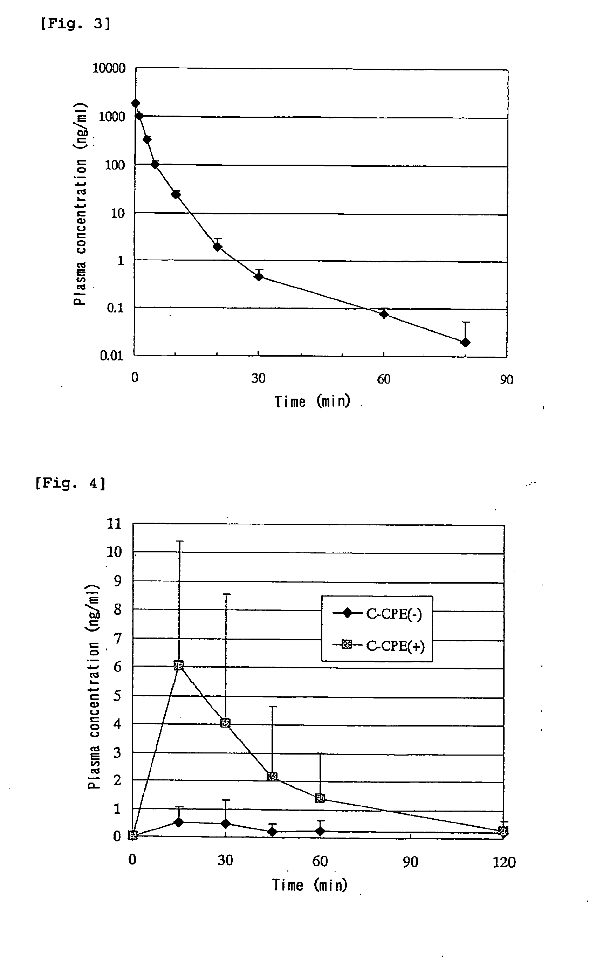

Fig. 3 is a graph showing changes in the plasma concentration after intravenous administration of human motilin to rats.

Fig. 4 is a graph showing changes in the plasma concentration of hPTH(1-34) in rats administered 100 μg/rat of hPTH(1-34) via the intestinal route. The line connecting solid diamonds corresponds to changes in the concentration observed when hPTH(1-34) was administered alone while the line connecting the solid squares corresponds to changes in the concentration observed when 20 μg of C-CPE were administered 4 hours before the administration of hPTH(1-34).

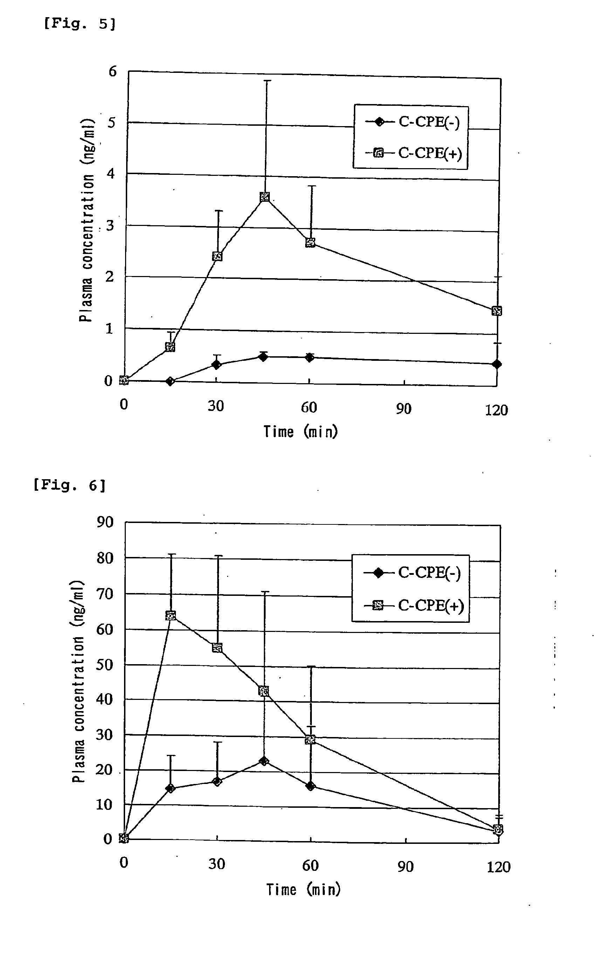

Fig. 5 is a graph showing changes in the plasma concentration of hPTH(1-34) in rats administered 200 μg/rat of hPTH(1-34) via the nasal route. The line connecting solid diamonds corresponds to changes in the concentration observed when hPTH(1-34) was administered alone while the line connecting the solid squares corresponds to changes in the concentration observed when 4 μg of C-CPE were administered 4 hours before the administration of hPTH(1-34).

Fig. 6 is a graph showing changes in the plasma concentration of hPTH(1-34) in rats administered 150 μg/rat of hPTH(1-34) through the pulmonary route. The line connecting solid diamonds corresponds to changes in the concentration observed when hPTH(1-34) was administered alone while the line connecting the solid squares corresponds to changes in the concentration observed when 4 μg of C-CPE were administered 4 hours before the administration of hPTH(1-34).

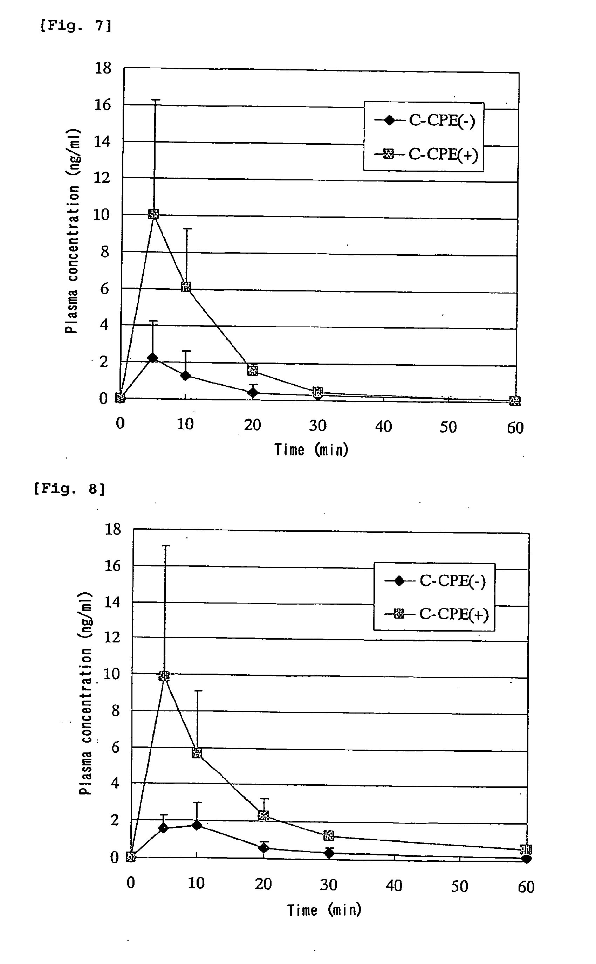

Fig. 7 is a graph showing changes in the plasma concentration of human ghrelin in rats administered 25 μg/rat of human ghrelin through the pulmonary route. The line connecting solid diamonds corresponds to changes in the concentration observed when human ghrelin was administered alone while the line connecting the solid squares corresponds to changes in the concentration observed when 5 μg of C-CPE were administered 4 hours before the administration of human ghrelin.

Fig. 8 is a graph showing changes in the plasma concentration of human motilin in rats administered 25 μg/rat of human motilin through the pulmonary route. The line connecting solid diamonds corresponds to changes in the concentration observed when human motilin was administered alone while the line connecting the solid squares corresponds to changes in the concentration observed when 5 μg of C-CPE were administered 4 hours before the administration of human motilin.

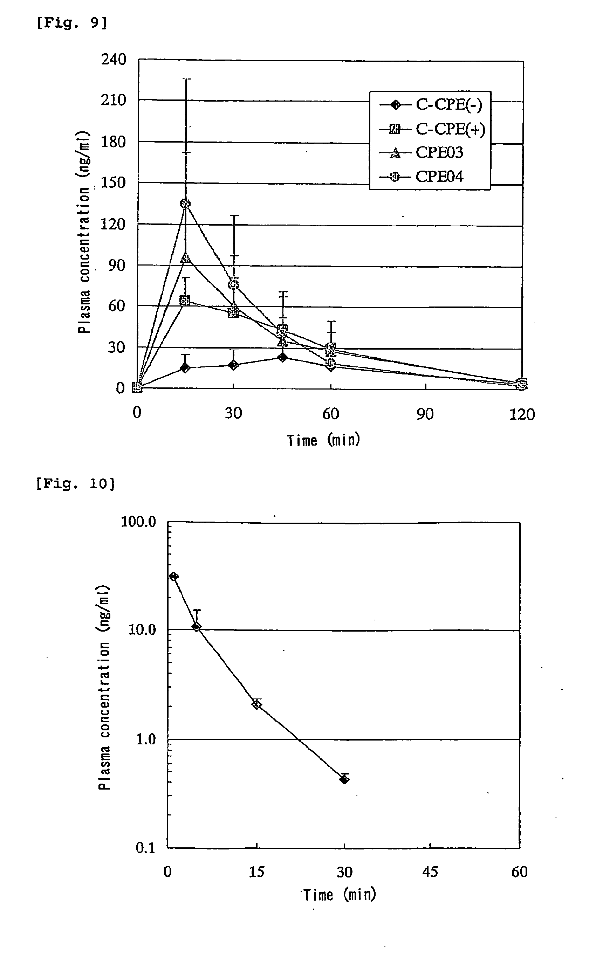

Fig. 9 is a graph showing changes in the plasma concentration of hPTH(1-34) in rats administered 150 μg/rat of hPTH(1-34) via the pulmonary route. The line connecting solid diamonds corresponds to changes in the concentration observed when hPTH(1-34) was administered alone and the line connecting the solid squares corresponds to changes in the concentration observed when 4 μg of C-CPE were administered 4 hours before the administration of hPTH(1-34). The line connecting solid triangles corresponds to changes in the concentration observed when 105 μg of CPE03 were administered 2 hours before the administration of hPTH(1-34) and the line connecting the solid circles corresponds to changes in the concentration observed when 20 μg of CPE04 were administered 2 hours before the administration of hPTH(1-34).

Fig. 10 is a graph showing changes in the plasma concentration after intravenous administration of hPTH(1-34) to rats.

## BEST MODE FOR CARRYING OUT THE INVENTION

The present invention will now be described in detail. C-CPE, the absorption-enhancing agent for use in the present invention, is C-terminal fragment (C-CPE) of an enterotoxin (CPE) produced by *Clostridium perfringens,* a bacterium belonging to the genus Clostridium. The amino acid sequence and the corresponding DNA base sequence of C-CPE are given by SEQ ID NO: 1 and SEQ ID NO: 2, respectively. The DNA base sequence of C-CPE was confirmed by the present inventor.

In addition to C-CPE, the mutants of the C-terminal fragment (C-CPE) that are functionally equivalent analogues of C-CPE and result from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE can also be used in the present invention.

Multiple articles report on the production of C-CPE mutants to analyze the structure of C-CPE. Some of these mutants are reported to lack the ability to enhance the intestinal mucosal absorption of dextran in rats or have a decreased ability to do so as compared to C-CPE. None of these C-CPE mutants has been reported to have higher ability to enhance mucosal absorption of dextran than does C-CPE, nor has any been reported to have the ability to enhance mucosal absorption of peptide drugs.

Kondoh et al. (Non-Patent Document 8 to 10) produced several amino acid-substituted mutants of C-CPE in an effort to find the binding center of C-CPE to claudin and conducted experiments to study their ability to enhance the intestinal mucosal absorption of dextran in fluorescently labeled rats. They report that some of these mutants did not enhance the absorption, while others had a decreased ability to enhance the absorption as compared to C-CPE or had a comparable ability to C-CPE.

Non-Patent Document 7 reports on the exemplary production of an N-terminal Δ36 mutant (36 residues have been deleted from the N-terminal) of C-CPE called C-CPE220 (CPE220-319). The report states that C-CPE220 did not have the ability to enhance intestinal mucosal absorption of dextran in rats.

Non-Patent Document 5 reports on the exemplary production of two C-CPE mutants C-CPE289 (CPE184-289) and C-CPE303 (CPE184-303) obtained by deletion of 30 and 16 residues from the C-terminal, respectively. The report states that neither of these C-CPE mutants had the ability to enhance intestinal mucosal absorption of dextran in rats.

Non-Patent Document 8 describes two C-CPE mutants Y306A and Y306K obtained by substitution of the Tyr residue at position 306 with an Ala residue and a Lys residue, respectively (i.e., point mutation of CPE184-319). It is reported that neither of these C-CPE mutants had the ability to enhance intestinal mucosal absorption of dextran in rats. The report also describes C-CPE mutants Y306F and Y306W obtained by substitution of the Tyr residue at position 306 with a Phe residue and a Trp residue, respectively. These mutants are reported to have a comparable ability to C-CPE to enhance intestinal mucosal absorption of dextran in rats.

Non-Patent Document 9 describes a triple mutant Y306A/Y310A/Y312A obtained by simultaneous substitution of the Tyr residue at position 306, the Tyr residue at position 310 and the Tyr residue at position 312, each with an Ala residue. The report states that the mutant did not have the ability to enhance the intestinal mucosal absorption in rats, and that the point mutants Y306A and Y312A, as well as the double mutants Y306A/Y310A, Y306A/Y312A and Y310A/Y312A, had a decreased ability to enhance the intestinal mucosal absorption in rats.

Non-Patent Document 10 describes a mutant L315A and a double mutant Y306A/L315A, each containing L315A and obtained by substitution of the Leu residue at position 315 with an Ala residue. Each mutant is reported to have a significantly decreased ability to enhance intestinal mucosal absorption in rats as compared to C-CPE.

Non-Patent Document 6 reports on the production of a mutant (CPE194-319) obtained by deletion of 10 amino acids from the N-terminal. CPE194-319 is a mutant constructed with the intension of increasing the solubility of C-CPE to thereby avoid aggregation of C-CPE and obtain the stable C-CPE protein for crystal structure analysis of C-CPE protein. Although Non-Patent Document 6 describes the solubility and stability in an aqueous solution of the mutant (CPE194-319) obtained by deletion of 10 amino acids from the N-terminal, as well as the binding activity of the mutant to claudin-4, nothing is mentioned or even suggested concerning its ability to enhance mucosal absorption of peptide drugs, which is the objective of the present invention.

As described above, while several preparing examples of different C-CPE mutants are known, it has not been known that the N-terminal deleted mutants of C-CPE retain the ability of C-CPE to enhance absorption, nor has it been known that the mutants have the ability to enhance mucosal absorption of peptide drugs - the ability claimed by the present invention.

Patent Document D1 discloses the production of *Clostridium perfringens* type A enterotoxin toxoids in E. coli for use in treating the symptoms of *Clostridium perfringens* food poisoning in patients.

Patent Document D2 discloses, *inter alia*, a method of treating ovarian cancer and uterine cancer by administering *Clostridium perfringens* enterotoxin.

Patent Document D3 provides new anti-cancer therapeutic agents involving ligands to the insuli-like growth factor-1 (IGF-1) receptor coupled to a therapeutic radionuclide or to a cellular toxin such as diphtheria toxin or Pseudomonas exotoxin.

Patent Document D5a (corresponding to Japanese Patent Document D5) discloses a pharmaceutical composition comprising a physiologically active peptide or protein and a heat-labile enterotoxin B subunit for increased absorption when administered through mucous.

The present inventors have constructed different C-CPE mutants based on their expected kinetics in the body and identified the C-CPE mutants of the present invention that have the ability to enhance mucosal absorption of peptide drugs.

The C-CPE mutants for use in the present invention are mutants resulting from the deletion in the C-terminal fragment (C-CPE). In the present invention, N-terminal-deleted mutants of the C-terminal fragment (C-CPE) are preferably used. Particularly preferred mutants are those obtained by deletion of one or several amino acid residues from amino acid residues 1 to 21 of the N-terminal of C-CPE.

Of these mutants, one mutant obtained by deletion of amino acid residues 1 to 10 (ERCVLTVPST) of the N-terminal of C-CPE is called "CPE03." The amino acid sequence of CPE03 is shown as SEQ ID NO: 3. The mutant CPE03 has an identical amino acid sequence to CPE194-319 described in Non-Patent Document 6.

A mutant obtained by deletion of amino acid residues 1 to 21 (ERCVLTVPSTDIEKEILDLAA) of the N-terminal of C-CPE is called "CPE04." The amino acid sequence of CPE04 is shown as SEQ ID NO: 4.

The C-CPE or its mutants for use in the present invention are the gene products that can be obtained using the toxin family obtained from *Clostridium perfringens* of the genus Clostridium or highly homologous toxin families obtained from the bacteria of the same genus, or chimeras of these toxins. Chimeras of highly homologous toxin proteins obtained from the bacteria of this genus are expected to provide similar effects.

The peptides of the C-CPE or its mutants for use in the present invention can be extracted from cultured bacterial cells of *Clostridium perfringens* or produced as recombinant proteins in host cells such as *E. coli* using genetic engineering techniques.

The gene products of the C-CPE or its mutants may be chemically modified such as by partial substitution of amino acid residues.

The C-CPE or its mutants for use in the present invention can be extracted from cultured bacterial cells of *Clostridium perfringens* or produced as recombinant proteins in host cells such as *E. coli* using genetic engineering techniques. His tag, GST tag or other purification-assisting tag peptides may be added to the N-terminal of C-CPE to simplify the purification process of C-CPE or the mutants of C-CPE.

Although the present invention can enhance mucosal permeation of various peptide drugs, the addition of C-CPE is particularly effective in enhancing mucosal absorption of parathyroid hormone and derivatives thereof, as well as of other peptide drugs including glucagon-like peptide-1, ghrelin, atrial natriuretic peptide, brain natriuretic peptide (BNP), C-type natriuretic peptide, insulin, motilin, leptin, resistin, glucagon, relaxin, galanin, gastrin, apelin, selectin, calcitonin, adrenomedullin, amylin, humanin, thymosin, endorphin, endomorphin, nocistatin, enkephalin, neuropeptide Y, neuropeptide S, neuromedin U, angiotensin, endothelin, guanylin, salusin, urotensin, oxytocin, vasopressin, neurophysin, melanocyte-stimulating hormone, urocortin, lipotropin, luteinizing hormone-releasing hormone, mystatin, prolactin-releasing peptide, somatostatin, cortistatin, thyrotropin-releasing hormone, substance P, neurokinin, endokinin, neurotensin, neoromedin N, obestatin, orexin, insulin-like growth factor-1 (IGF-1), melanin-concentrating hormone, corticotropin-releasing hormone, exendin-4, catacalcin, cholecystokinin, corticotrophin, melanotrophin, neoromedin C, copeptin, pituitary adenylate cyclase-activating peptide (PACAP), peptide YY, thyroliberin and derivatives thereof, and peptide derivatives including non-natural amino acids or chemically modification. More preferably, the peptide drugs have a molecular weight of approximately 20,000 or less.

The C-CPE or its mutants to serve as the mucosal absorption-enhancing agent of the present invention can be used in combination with a peptide drug to enhance the biological absorption of the peptide drug.

With regard to the timing for administering the C-CPE or its mutants with the peptide drug, the C-CPE or its mutants may be co-administered with the peptide drug to achieve the desired ability to enhance mucosal absorption of the peptide drug. When the mucosal absorption-enhancing agent may be administered and the tight junctions sufficiently open in advance to administrate of peptide drug, thus the peptide drug can quickly permeate the space between cells and the drug is exposed to digestion by proteases secreted by the mucosal epithelium cells for a decreased time period, resulting in further enhancement of the biological absorption of the peptide drug. Thus, it is preferred to administer the C-CPE or its mutants to serve as the mucosal absorption-enhancing agent of the present invention before the administration of the peptide drug.

It is desirable that the mucosal absorption-enhancing agent be administered approx. 15 minutes to approx. 24 hours before the administration of the peptide drug to permit sufficient time to allow the C-CPE or its mutants being the mucosal absorption-enhancing agent to open the tight junctions. Studies conducted by the present inventors have revealed that the mucosal absorption is significantly enhanced by administering the C-CPE or its mutants at least two hours before the administration of the peptide drug.

In addition to direct administration, the C-CPE or its mutants for use with the peptide drug may be administered in various forms. For example, the C-CPE or its mutants may be incorporated in an outer layer of capsules composed of core tablets containing the peptide drug and an enteric polymer coating.

An oral capsule may also be used that is composed of an inner core encapsulating the desired peptide drug, and an inner membrane formed of an enteric polymer, which encloses the inner core and is designed to disintegrate in a delayed manner. In such a case, an outer shell polymer first dissolves to release the C-CPE or its mutants to open the tight junctions in the epithelium of the intestinal mucosa. Subsequently, the inner membrane polymer disintegrates to release the peptide drug encapsulated therein. As a result, the peptide drug is effectively absorbed by the living body through the opened tight junctions.

When it is desired to co-administer the C-CPE or its mutants with the peptide, the two components may be simply mixed together. Also, the two components may be crosslinked by chemical modification. Alternatively, a primary sequence of amino acids containing the C-CPE or its mutants and the peptide drug to be administered directly linked to the N-terminal or the C-terminal of the C-CPE or its mutants may be biosynthesized and administered.

The two components in the primary sequence may be linked either directly to each other or indirectly via a linker sequence that is recognized and cleaved by trypsin or other proteases localized in the blood or epithelium of mucosa.

The composition for mucosal administration, the mucosal absorption-enhancing agent and the peptide drug provided by the present invention may be provided in various forms that are designed to suit the desired purpose of treatment. Specific examples include tablets, pills, powders, solutions, suspensions, emulsions, granules, capsules and suppositories. Powders, aqueous suspensions and oil suspensions are particularly suitable for the administration of the composition for mucosal administration.

A typical mucosa, the administration site to which the composition for mucosal administration of the present invention is administered to enhance biological absorption, is small intestinal mucosal epithelial tissue that can be studied using Caco-2 cell line model. Similar effects are expected in other mucosal epithelial tissue in which claudin-4 is expressed at high levels. Examples of such tissue include epithelial tissue of nasal mucosa, respiratory tract mucosa, lung mucosa, vaginal mucosa, eye mucosa, oral mucosa and rectal mucosa. CPE is known to act also on claudins-3, -6, -7, - 8 and -14 although its binding activity to these claudins is lower than to claudin-4. Thus, CPE is expected to provide similar effects in any type of tissue expressing these members of the claudin family.

The dose of the C-CPE or its mutants to be administered is not limited to a particular range of dose and may vary depending on the dose of the peptide drug used therewith, the type of administration site in which the peptide drug is to be absorbed from the mucosa and other factors. The C-CPE or its mutants is desirably administered in a sufficient dose to open the tight junctions in the mucosal epithelial cell layer. Specifically, it is preferred that the C-CPE or its mutants be administered in a single dose of 0.1 µg to 100 µg.

The above-described invention makes it possible to enhance mucosal absorption of peptide drugs through intestinal, pulmonary and nasal routes by allowing the peptide drugs in combination with the C-terminal fragment (C-CPE) of an enterotoxin (CPE) produced by the bacterium *Clostridium perfringens* of the genus Clostridium, or mutants of C-CPE to act thereon. Thus, the composition for mucosal administration provided in accordance with the present invention enhances biological absorption of peptide drugs, such as hPTH(1-34), human ghrelin and human motilin, through the mucosa of small intestine, lung, nasal cavity and other mucosa. The enhancement of biological absorption by the composition for mucosal administration of the present invention is significant. Also, unlike any of the conventional mucosal absorption-enhancers, the composition for mucosal administration of the present invention does not cause tissue damage and is therefore highly safe for use and enables highly effective absorption of the peptide drugs by a living body.

EXAMPLES

The present invention will now be described in specific details with reference to examples and comparative examples, which are not intended to limit the scope of the invention in any way.

Example 1: Preparation of C-CPE

A C-terminal fragment (amino acid residues 184 - 319) of enterotoxin cloned from the strain NCTC8239 of Clostridium perfringens, also known as Clostridium welchii (Health Protection Agency, The National Collection of Type Cultures, London, UK) was integrated into the plasmid pET16b, a plasmid designed to express His-tagged fusion protein (Novagen Inc., Madison, WI, USA), to construct expression plasmid $pETH_{10}PER$ (J. Cell Biol. Vol. 136, 1239-47). The expression plasmid was transfected into E. coli strain BL21 and the cells were cultured in LB medium supplemented with ampicillin.

IPTG was added to induce expression of His-tagged fusion protein and the cells were collected by centrifugation and lysed by sonication. The lysate was centrifuged at 15000 rpm for 15 minutes and the supernatant was collected and loaded on a Ni-chelate column.

The column was washed with 10 mM Tris-HCl buffer (pH 8.0) containing 200 mM imidazole and the His-tagged fusion protein was eluted with 10 mM Tris-HCl buffer (pH 8.0) containing 400 mM imidazole. The eluate was replaced with PBS buffer (pH 7.4) on a gel filtration column and was used as a sample for evaluating the ability to enhance mucosal absorption.

Example 2: Preparation of C-CPE mutant

DNA base sequences encoding 10 amino acid residues from the N-terminal of C-CPE and 21 amino acid residues from the N-terminal of C-CPE were each excised from $pETH_{10}PER$ to construct expression plasmids pCPE03 and pCPE04, respectively, for expressing Δ10aa C-CPE (CPE03), having 10 residues deleted from the N-terminal of C-CPE, and Δ21aa C-CPE (CPE04), having 21 residues deleted from the N-terminal of C-CPE, in E. coli. Each expression plasmid was transfected into E. coli strain BL 21 and the cells were cultured in LB medium supplemented with ampicillin. IPTG was added to induce expression of His-tagged fusion protein and the cells were collected by centrifugation and lysed by sonication. The lysate was centrifuged at 15000 rpm for 15 minutes and the supernatant was collected and loaded on a Ni-chelate column. The column was washed with 10mM Tris-HCl buffer (pH 8.0) containing 200 mM imidazole and the His-tagged fusion protein was eluted with 10 mM Tris-HCl buffer (pH 8.0) containing 400 mM imidazole. The eluate was replaced with PBS buffer (pH 7.4) on a gel filtration column and the resulting CPE03 and CPE04 were used as samples for evaluating their ability to enhance mucosal absorption.

Comparative Example 1: Pharmacokinetics of after subcutaneous administration of hPTH(1-34) to rats

A solution of human parathyroid hormone hPTH(1-34) was subcutaneously administered to rats and the plasma concentration was measured.

Subcutaneous administration was performed on rats having a polyethylene tube (PE-50, Clay Adams) inserted into the femoral artery. Five- to six-week-old male SD rats (Charles River Laboratories Japan) were divided into groups of 5 animals and were used in the experiment. hPTH(1-34) was dissolved in physiological saline containing 0.1% BSA to form a 10 $\mu$g/ml solution. Using a syringe and a 26G injection needle (each manufactured by Terumo), this solution was subcutaneously injected into the back skin in a dose of 1 mL/kg. Blood samples were collected from the polyethylene tube inserted into the femoral artery before the administration and 5, 15, 30, 45, 60, 90 and 120 minutes after the administration.

To the collected blood samples, one-hundredth by volume of EDTA·2Na·2H$_2$O solution was immediately added and the samples were centrifuged to separate the plasma fraction. One-tenth by volume of 5000 IU/mL aprotinin solution was immediately added to the plasma and the solution was mixed and stored at -80°C until measurement.

The plasma concentration of hPTH(1-34) was measured by radioimmunoassay (RIA) using anti-hPTH antibody. Specifically, anti-hPTH antibody and [$^{125}$I-Tyr$^{34}$]hPTH(1-34) were sequentially added to the plasma sample for competitive immunoreaction. To this sample, secondary antibody was added to precipitate hPTH(1-34) bound to the anti-hPTH antibody. After separation of the supernatant, the radioactivity of the precipitated fraction was measured by a $\gamma$-counter (Packard). The resulting plasma concentration of hPTH(1-34) over time is shown in Fig. 1.

Comparative Example 2: Pharmacokinetics after intestinal administration of hPTH(1-34) to rats

Intestinal administration was also performed on rats with a polyethylene tube inserted into the femoral artery. Specifically, hPTH(1-34) solution was administered into the jejunum of rats using *in situ* loop technique. Seven-week-old male Wister rats (Charles River Laboratories Japan) were divided into groups of 6 animals and were used in the experiment. Using a surgical suture, jejunum was loosely ligated immediately below the opening of the common bile duct and 5cm distal to the first ligation to form a loop. Using scissors, an incision was made at both ends of the resulting loop. 50 mL of PBS (pH 6.5) was injected from the incision to wash the lumen and the distal end was ligated. hPTH (1-34) was dissolved in PBS (pH 6.5) to form a 0.5 mg/mL solution. Using a syringe and an 18G injection needle (each manufactured by Terumo), the solution was administered in a dose of 0.2 mL/rat from the proximal end of the loop. The proximal end of the loop was then ligated and the jejunum was returned to the abdominal cavity. Blood samples were collected from the polyethylene tube inserted into the femoral artery before administration and 15, 30, 45, 60 and 120 minutes after administration. The collected blood samples were treated in the same manner as in Comparative Example 1 to separate the plasma. The plasma concentration of hPTH(1-34) was measured by RIA.

Comparative Example 3: Pharmacokinetics after intranasal administration of hPTH(1-34) to rats

Intranasal administration was also performed on rats with a polyethylene tube inserted into the femoral artery. Seven-week-old male SD rats (Charles River Laboratories Japan) were divided into groups of 3 animals and were used in the experiment. hPTH(1-34) was dissolved in PBS (pH 6.5) to form a 10 mg/mL solution. 10 $\mu$L of the solution was administered to each nasal cavity of rats (20 $\mu$L in total). Blood samples were collected from the polyethylene tube inserted into the femoral artery before administration and 15, 30, 45, 60 and 120 minutes after administration. The collected blood samples were treated in the same manner as in Comparative Example 1 to separate the plasma. The plasma concentration of hPTH(1-34) was then measured by RIA.

Comparative Example 4: Pharmacokinetics after pulmonary administration of hPTH(1-34) to rats

Pulmonary administration was also performed on rats with a polyethylene tube inserted into the femoral artery. Specifically, hPTH(1-34) solution was directly administered into the trachea. Seven-week-old male SD rats (Charles River Laboratories Japan) were divided into groups of 6 animals and were used in the experiment. A polyethylene tube (PE-240, Clay Adams) was inserted into the trachea of rats prior to administration. hPTH(1-34) was dissolved in PBS (pH 6.5) to form a 10 mg/mL solution. Using a tracheal liquid-spraying apparatus (MicroSprayer, Penn Century), 15 $\mu$L of the solution was administered into the polyethylene tube inserted in the trachea. Blood samples were collected from the polyethylene tube inserted into the femoral artery before administration and 15, 30, 45, 60 and 120 minutes after administration. The collected blood samples were treated in the same manner as in Comparative Example 1 to separate the plasma. The plasma concentration of hPTH(1-34) was then measured by RIA.

As the pharmacokinetic parameters, the maximum plasma concentration ($C_{max}$) and the area under the plasma concentration-time curve (AUC) were calculated from the change in the plasma concentration of hPTH(1-34) over time, as determined in the above-described tests. $C_{max}$ was determined from the actual measurements and AUC was determined by the trapezoidal method. These values were used in the following equation to calculate the bioavailability (BA):

$$BA(\%) = (AUC/Dose)/(AUC(sc)/Dose(sc))$$

where
AUC = AUC (ng•min/mL) after intestinal, intranasal or pulmonary administration;
Dose = dose ($\mu$g/kg) administered through intestinal, nasal or pulmonary route;
AUC (sc) = AUC (ng•min/mL) after subcutaneous administration; and
Dose (sc) = dose ($\mu$g/kg) administered through subcutaneous route.

These results are shown in Table 1. The values are averages of 3 to 6 rats in the respective groups.

Table 1: Pharmacokinetic parameters after subcutaneous, intestinal, intranasal or pulmonary administration of hPTH(1-34) to rats.

[Table 1]

| Administration route | Dose | | $C_{max}$ (ng/mL) | AUC (ng·min/mL) | BA (%) |
|---|---|---|---|---|---|
| | (μg/rat) | (μg/kg) | | | |
| Subcutaneous administration | - | 10 | 1.07 ± 0.41 | 49.6 ± 28.0 | - |
| Intestinal administration | 100 | 378.0 ± 36.8 | 0.93 ± 0.80 | 32.3 ± 30.2 | 1.8 ± 1.8 |
| Intranasal administration | 200 | 822.2 ± 19.2 | 0.67 ± 0.16 | 43.9 ± 11.6 | 1.1 ± 0.3 |
| Pulmonary administration | 150 | 515.7 ± 67.0 | 27.84 ± 15.90 | 1534.1 ± 1103.0 | 61.3 ± 45.0 |

As can be seen from the results shown in Table 1, the hPTH(1-34) solution administered through the subcutaneous route in a dose of 10 μg/kg gave an AUC of 49.6 ng·min/mL. The hPTH(1-34) solution administered through the intestinal route in a dose of 100 μg/rat gave an AUC of 32.3 ng•min/mL. The hPTH(1-34) solution administered through the nasal route in a dose of 200 μg/rat gave an AUC of 43.9 ng·min/mL. The hPTH(1-34) solution administered through the pulmonary route in a dose of 150 μg/rat gave an AUC of 1534.1 ng· min/mL. Thus, the BA values of hPTH (1-34) administered through intestinal, nasal or pulmonary route were determined to be 1.8%, 1.1% and 61.3%, respectively.

Comparative Example 5: Pharmacokinetics after intravenous administration of human ghrelin to rats

A solution of human ghrelin (hGhrelin) was intravenously administered to rats and the plasma concentration was measured.

Intravenous administration was performed on rats having a polyethylene tube (PE-50, Clay Adams) inserted into the femoral artery. Seven-week-old male SD rats (Charles River Laboratories Japan) were divided into groups of 5 animals and were used in the experiment. Human ghrelin was dissolved in a 5% mannitol solution to form a 20 μg/ml solution. Using a syringe and a 26G injection needle (each manufactured by Terumo), this solution was injected into the tail vein in a dose of 0.5 mL/kg. Blood samples were collected from the polyethylene tube inserted into the femoral artery before administration and 1, 3, 5, 10, 20, 30, 60 and 90 minutes after administration.

To the collected blood samples, one-hundredth by volume of EDTA·2Na·2H$_2$O solution and one-fiftieth by volume of 4-(2-aminoethyl)-benzenesulfonyl fluoride hydrochloride (AEBSF) solution were immediately added and the samples were centrifuged to separate the plasma fraction. One-tenth by volume of 1N hydrochloric acid was immediately added to the plasma and the solution was mixed and stored at -80°C until measurement.

The plasma concentration of human ghrelin was measured by radioimmunoassay (RIA) using anti-ghrelin antibody. Specifically, anti-hPTH antibody and [$^{125}$I-Tyr[29]]human ghrelin were sequentially added to the plasma sample for competitive immunoreaction. To this sample, secondary antibody was added to precipitate human ghrelin bound to the anti-human ghrelin antibody. After separation of the supernatant, the radioactivity of the precipitated fraction was measured by a γ-counter (Perkin Elmer Co., Ltd.). The resulting plasma concentration of human ghrelin over time is shown in Fig. 2.

As the pharmacokinetic parameter, the area under the plasma concentration-time curve (AUC) was calculated from the change in the plasma concentration of human ghrelin over time. C0 was determined by extrapolation and AUC was determined by the trapezoidal method. The human ghrelin solution administered through the intravenous route in a dose of 10 μg/kg gave a C0 of 40.69 ng/mL and an AUC of 206.0 ng·min/mL.

Comparative Example 6: Pharmacokinetics after pulmonary administration of human ghrelin to rats

Human ghrelin solution was directly administered into the trachea of rats having a polyethylene tube inserted into the femoral artery. Seven-week-old male SD rats (Charles River Laboratories Japan) were divided into groups of 6 animals and were used in the experiment. A polyethylene tube (PE-240, Clay Adams) was inserted into the trachea of rats prior to administration. Human ghrelin was dissolved in PBS (pH 6.5) to form a 1 mg/mL solution. Using a tracheal liquid-spraying apparatus (MicroSprayer, Penn Century), 25 μL of the solution was administered into the polyethylene tube inserted in the trachea. Blood samples were collected from the polyethylene tube inserted into the femoral artery before administration and 5, 10, 20, 30 and 60 minutes after administration. The collected blood samples were treated in the same manner as in Comparative Example 5 to separate the plasma. The plasma concentration of human ghrelin was then measured by RIA.

Comparative Example 7: Pharmacokinetics after intravenous administration of human motilin to rats

A solution of human motilin (hMotilin) was intravenously administered to rats and the plasma concentration was measured.

Intravenous administration was performed on rats having a polyethylene tube (PE-50, Clay Adams) inserted into the femoral artery. Seven-week-old male SD rats (Charles River Laboratories Japan) were divided into groups of 5 animals and were used in the experiment. Human motilin was dissolved in a 5% mannitol solution to form a 100 $\mu$g/ml solution. Using a syringe and a 26G injection needle (each manufactured by Terumo), this solution was injected into the tail vein in a dose of 1 mL/kg. Blood samples were collected from the polyethylene tube inserted into the femoral artery before administration and 1, 3, 5, 10, 20, 30, 60 and 80 minutes after administration.

To the collected blood samples, one-hundredth by volume of EDTA·2Na·2H$_2$O solution was immediately added and the samples were centrifuged to separate the plasma fraction. One-tenth by volume of 5000 IU/mL aprotinin solution was immediately added to the plasma and the solution was mixed and stored at -80°C until measurement.

The plasma concentration of human motilin was measured by radioimmunoassay (RIA) using anti-human motilin antibody. Specifically, anti-human motilin antibody and $^{125}$I-human motilin were sequentially added to the plasma sample for competitive immunoreaction. To this sample, secondary antibody was added to precipitate human motilin bound to the anti-human motilin antibody. After separation of the supernatant, the radioactivity of the precipitated fraction was measured by a $\gamma$-counter (Perkin Elmer Co., Ltd.). The resulting plasma concentration of human motilin over time is shown in Fig. 3.

As the pharmacokinetic parameter, the area under the plasma concentration-time curve (AUC) was calculated from the change in the plasma concentration of human motilin over time. C0 was determined by extrapolation and AUC was determined by the trapezoidal method. The human motilin solution administered through the intravenous route in a dose of 100 $\mu$g/kg gave a C0 of 1797 ng/mL and an AUC of 3598 ng·min/mL.

Comparative Example 8: Pharmacokinetics after pulmonary administration of human motilin to rats

Human motilin solution was directly administered into the trachea of rats having a polyethylene tube inserted into the femoral artery. Seven-week-old male SD rats (Charles River Laboratories Japan) were divided into groups of 3 animals and were used in the experiment. A polyethylene tube (PE-240, Clay Adams) was inserted into the trachea of rats prior to administration. Human motilin was dissolved in PBS (pH 6.5) to form a 1 mg/mL solution. Using a tracheal liquid-spraying apparatus (MicroSprayer, Penn Century), 25 $\mu$L of the solution was administered into the polyethylene tube inserted in the trachea. Blood samples were collected from the polyethylene tube inserted into the femoral artery before administration and 5, 10, 20, 30 and 60 minutes after administration. The collected blood samples were treated in the same manner as in Comparative Example 7 to separate the plasma. The plasma concentration of human motilin was then measured by RIA.

Example 3: Ability of C-CPE to enhance absorption after intestinal administration of hPTH(1-34) to rats

Seven-week-old male Wister rats (Charles River Laboratories Japan) were divided into groups of 6 animals and were used in the experiment. A jejunal loop was formed in the same manner as in Comparative Example 2 and a 0.1 mg/mL solution of C-CPE was administered in a dose of 0.2 mL/rat from the proximal end of the loop. Four hours after administration of C-CPE, a 0.5 mg/mL solution of hPTH(1-34) was administered in the loop in a dose of 0.2 mL/rat. The plasma concentration of hPTH(1-34) was measured by RIA. The results are shown in Fig. 4. The pharmacokinetic parameters are shown in Table 2 below.

Table 2: Pharmacokinetic parameters after intestinal administration of hPTH(1-34) to rats (Enhanced absorption by C-CPE).

[Table 2]

| C-CPE | Dose | | C$_{max}$ (ng/mL) | AUC (ng·min/mL) | BA (%) |
|---|---|---|---|---|---|
| | ($\mu$g/rat) | ($\mu$g/kg) | | | |
| - | 100 | 378.0 $\pm$ 36.8 | 0.93 $\pm$ 0.80 | 32.3 $\pm$ 30.2 | 1.8 $\pm$ 1.8 |
| + | 100 | 403.8 $\pm$ 57.5 | 6.04 $\pm$ 4.34 | 243.6 $\pm$ 229.0 | 11.3 $\pm$ 8.9 |

As can be seen from the results shown in Table 2, the BA value of hPTH(1-34) was 11.3% with the intestinal administration of C-CPE 4 hours before the administration of hPTH(1-34). This means that, surprisingly, the BA of hPTH(1-34) preceded by administration of C-CPE was 6.3 times higher than the BA for hPTH(1-34) alone (1.8%), showing a marked increase.

Example 4: Ability of C-CPE to enhance absorption after intranasal administration of hPTH(1-34) to rats

Seven-week-old male SD rats (Charles River Laboratories Japan) were divided into groups of 3 animals and were used in the experiment. 10 μL of a 0.2 mg/mL solution of C-CPE was administered to each nasal cavity of rats (20 μL in total). Four hours after administration of C-CPE, 10 μL of a 10 mg/mL solution of hPTH(1-34) was administered to each nasal cavity of rats (20 μL in total). The plasma concentration of hPTH(1-34) was measured by RIA. The results are shown in Fig. 5. The pharmacokinetic parameters are shown in Table 3 below.
Table 3: Pharmacokinetic parameters after intranasal administration of hPTH(1-34) to rats (Enhanced absorption by C-CPE).
[Table 3]

| C-CPE | Dose | | $C_{max}$ (ng/mL) | AUC (ng·min/mL) | BA (%) |
|---|---|---|---|---|---|
| | (μg/rat) | (μg/kg) | | | |
| - | 200 | 822.2 ± 19.2 | 0.67 ± 0.16 | 43.9 ± 11.6 | 1.1 ± 0.3 |
| + | 200 | 833.3 ± 0.0 | 3.58 ± 2.30 | 244.5 ± 109.5 | 5.9 ± 2.6 |

As can be seen from the results shown in Table 3, the BA value of hPTH(1-34) with the intranasal administration of C-CPE 4 hours before the administration of hPTH(1-34) was 5.9%, which was 5.4 times higher than the BA for hPTH(1-34) alone. This indicates that the pre-administration of C-CPE also markedly increased the BA of hPTH(1-34) administered through the nasal route.

Example 5: Ability of C-CPE to enhance absorption after pulmonary administration of hPTH(1-34) to rats

Seven-week-old male SD rats (Charles River Laboratories Japan) were divided into groups of 6 animals and were used in the experiment. A polyethylene tube (PE-240, Clay Adams) was inserted into the trachea as in Comparative Example 4. Using MicroSprayer (Penn-Century, Inc.), a 0.2 mg/mL solution of C-CPE was administered in a dose of 20 μL/rat. Four hours after administration of C-CPE, a 10 mg/mL solution of hPTH(1-34) was administered in the trachea in a dose of 15 μL/rat. The plasma concentration of hPTH(1-34) was measured by RIA. The results are shown in Fig. 6. The pharmacokinetic parameters are shown in Table 4 below.
Table 4: Pharmacokinetic parameters after pulmonary administration of hPTH(1-34) to rats (Enhanced absorption by C-CPE).
[Table 4]

| C-CPE | Dose | | $C_{max}$ (ng/mL) | AUC (ng·min/mL) | BA (%) |
|---|---|---|---|---|---|
| | (μg/rat) | (μg/kg) | | | |
| - | 150 | 515.7 ± 67.0 | 27.84 ± 15.90 | 1534.1 ± 1103.0 | 61.3 ± 45.0 |
| + | 150 | 498.7 ± 50.3 | 67.63 ± 17.87 | 3631.8 ± 1816.9 | 145.1 ± 68.5 |

As can be seen from the results shown in Table 4, the BA value of hPTH(1-34) with the pulmonary administration of C-CPE 4 hours before the administration of hPTH(1-34) was 145.1%, which was 2.4 times higher than the BA for hPTH(1-34) alone. This indicates that the pre-administration of C-CPE also markedly increased the BA of hPTH(1-34) administered through the pulmonary route.

Example 6: Ability of C-CPE to enhance absorption after pulmonary administration of human ghrelin to rats

Human ghrelin solution was directly administered into the trachea of rats having a polyethylene tube inserted into the femoral artery. Seven-week-old male SD rats (Charles River Laboratories Japan) were divided into groups of 6 animals and were used in the experiment. The polyethylene tube (PE-240, Clay Adams) was inserted into the trachea as in Comparative Example 6. Using MicroSprayer (Penn-Century, Inc.), a 0.2 mg/mL solution of C-CPE was administered in a dose of 25 $\mu$L/rat. Four hours after administration of C-CPE, a 1 mg/mL solution of human ghrelin was administered in the trachea in a dose of 25 $\mu$L/rat. The plasma concentration of human ghrelin was measured by RIA. The results are shown in Fig. 7. As the pharmacokinetic parameters, the maximum plasma concentration ($C_{max}$) and the area under the plasma concentration-time curve (AUC) were calculated from the change in the plasma concentration of human ghrelin over time, as determined in the above-described tests of Comparative Example 6 and Example 6. $C_{max}$ was determined from the actual measurements and AUC was determined by the trapezoidal method. These values were used in the following equation to calculate the bioavailability (BA):

$$BA(\%) = (AUC/Dose)/(AUC(iv)/Dose(iv))$$

where
AUC = AUC (ng·min/mL) after pulmonary administration;
Dose = dose ($\mu$g/kg) administered through pulmonary route;
AUC (iv) = AUC (ng·min/mL) after intravenous administration; and
Dose (iv) = dose ($\mu$g/kg) administered through intravenous route.
The results are shown in Table 5.

Table 5: Pharmacokinetic parameters after pulmonary administration of human ghrelin to rats (Enhanced absorption by C-CPE).

[Table 5]

| C-CPE | Dose | | $C_{max}$ (ng/mL) | AUC (ng·min/mL) | BA (%) |
|---|---|---|---|---|---|
| | ($\mu$g/rat) | ($\mu$g/kg) | | | |
| - | 25 | 94.3 ± 8.0 | 2.25 ± 2.00 | 32.0 ± 30.3 | 1.6 ± 1.4 |
| + | 25 | 93.7 ± 7.8 | 10.07 ± 6.18 | 120.5 ± 60.9 | 6.1 ± 2.9 |

As can be seen from the results shown in Table 5, the BA value of human ghrelin with the pulmonary administration of C-CPE 4 hours before the administration of human ghrelin was 6.1%, which was 3.8 times higher than the BA for human ghrelin alone. This indicates that the pre-administration of C-CPE also markedly increased the BA of human ghrelin administered through the pulmonary route.

Example 7: Ability of C-CPE to enhance absorption after pulmonary administration of human motilin to rats

Human motilin solution was directly administered into the trachea of rats having a polyethylene tube inserted into the femoral artery. Seven-week-old male SD rats (Charles River Laboratories Japan) were divided into groups of 3 animals and were used in the experiment. The polyethylene tube (PE-240, Clay Adams) was inserted into the trachea as in Comparative Example 8. Using MicroSprayer (Penn-Century, Inc.), a 0.2 mg/mL solution of C-CPE was administered in a dose of 25 $\mu$L/rat. Four hours after administration of C-CPE, a 1 mg/mL solution of human motilin was administered in the trachea in a dose of 25 $\mu$L/rat. The plasma concentration of human motilin was measured by RIA. The results are shown in Fig. 8. The pharmacokinetic parameters are shown in Table 6 below.

Table 6: Pharmacokinetic parameters after pulmonary administration of human motilin to rats (Enhanced absorption by C-CPE).

[Table 6]

| C-CPE | Dose | | $C_{max}$ (ng/mL) | AUC (ng·min/mL) | BA (%) |
|---|---|---|---|---|---|
| | ($\mu$g/rat) | ($\mu$g/kg) | | | |
| - | 25 | 87.3 ± 3.4 | 1.89 ± 1.10 | 35.0 ± 20.9 | 1.1 ± 0.7 |
| + | 25 | 89.4 ± 3.2 | 9.85 ± 7.24 | 147.7 ± 80.5 | 4.5 ± 2.3 |

As can be seen from the results shown in Table 6, the BA value of human motilin with the pulmonary administration of C-CPE 4 hours before the administration of human motilin was 4.5%, which was 4.1 times higher than the BA for human motilin alone. This indicates that the pre-administration of C-CPE also markedly increased the BA of human motilin administered through the pulmonary route.

Example 8: Ability of C-CPE mutant to enhance absorption after pulmonary administration of hPTH(1-34) to rats

Seven-week-old male SD rats (Charles River Laboratories Japan) were divided into groups of 5 or 6 animals and were used in the experiment. A polyethylene tube (PE-240, Clay Adams) was inserted into the trachea as in Comparative Example 4. Using MicroSprayer (Penn-Century, Inc.), 4.2 mg/mL and 0.8 mg/mL solutions of C-CPE mutants CPE03 (having the amino acid sequence of SEQ ID NO: 3) and CPE04 (having the amino acid sequence of SEQ ID NO: 4) were administered, respectively, each in a dose of 20 $\mu$L/rat. Two hours after the administration of CPE03 and CPE04, a 10 mg/mL solution of hPTH(1-34) was administered in the trachea in a dose of 15 $\mu$L/rat. The plasma concentration of hPTH(1-34) was measured by RIA. The results are shown in Fig. 9. The pharmacokinetic parameters are shown in Table 7 below.

Table 7: Pharmacokinetic parameters after pulmonary administration of hPTH(1-34) to rats (Enhanced absorption by CPE03 and CPE04).

[Table 7]

| C-CPE | Dose | | $C_{max}$ (ng/mL) | AUC (ng·min/mL) | BA (%) |
|---|---|---|---|---|---|
| | ($\mu$g/rat) | ($\mu$g/kg) | | | |
| - | 150 | 515.7 $\pm$ 67.0 | 27.84 $\pm$ 15.90 | 1534.1 $\pm$ 1103.0 | 61.3 $\pm$ 45.0 |
| + | 150 | 498.7 $\pm$ 50.3 | 67.63 $\pm$ 17.87 | 3631.8 $\pm$ 1816.9 | 145.1 $\pm$ 68.5 |
| CPE03 | 150 | 597.5 $\pm$ 33.0 | 96.2 $\pm$ 76.38 | 4040.9 $\pm$ 2190.3 | 138.8 $\pm$ 78.7 |
| CPE04 | 150 | 608.1 $\pm$ 49.2 | 134.77 $\pm$ 91.39 | 4523.6 $\pm$ 3041.7 | 149.7 $\pm$ 98.9 |

As can be seen from the results shown in Table 7, the BA values of hPTH(1-34) with the pulmonary administration of CPE03 and CPE04 2 hours before the administration of hPTH(1-34) were 138.8% and 149.7%, respectively, which were 2.3 times and 2.4 times higher than the BA for hPTH(1-34) alone, respectively. This indicates that the pre-administration of the C-CPE mutants also markedly increased the BA of hPTH(1-34) administered through the pulmonary route.

Comparative Example 9: Pharmacokinetics after intravenous administration of hPTH(1-34) to rats

An hPTH(1-34) solution was intravenously administered to rats and the plasma concentration of hPTH(1-34) was measured.

Intravenous administration was performed on rats having a polyethylene tube (PE-50, Clay Adams) inserted into the femoral artery. Six- to 7-week-old male SD rats (Charles River Laboratories Japan) were divided into groups of 3 animals and were used in the experiment. hPTH(1-34) was dissolved in physiological saline containing 0.1% BSA to form a 10 $\mu$g/ml solution. Using a syringe and a 26G injection needle (each manufactured by Terumo), this solution was intravenously injected into the jugular vein in a dose of 1mL/kg. Blood samples were collected from the polyethylene tube inserted into the femoral artery before administration and 1, 5, 15, 30, 90, 120, 180 and 240 minutes after administration.

To the collected blood samples, one-hundredth by volume of EDTA·2Na·2H$_2$O solution was immediately added and the samples were centrifuged to separate the plasma fraction. One-tenth by volume of 5000 IU/mL aprotinin solution was immediately added to the plasma and the solution was mixed and stored at -80°C until measurement.

The plasma concentration of hPTH(1-34) was measured by radioimmunoassay (RIA) using anti-hPTH antibody. Specifically, anti-hPTH antibody and [$^{125}$I-Tyr$^{34}$]hPTH(1-34) were sequentially added to the plasma sample for competitive immunoreaction. To this sample, secondary antibody was added to precipitate hPTH(1-34) bound to the anti-hPTH antibody. After separation of the supernatant, the radioactivity of the precipitated fraction was measured by a $\gamma$-counter (Packard). The resulting plasma concentration of hPTH(1-34) over time is shown in Fig. 10.

As the pharmacokinetic parameters, the maximum plasma concentration ($C_{max}$) and the area under the plasma concentration-time curve (AUC) were calculated from the change in the plasma concentration of hPTH(1-34) over time, as determined in the above-described tests of Examples 2 to 4 and Comparative Example 9. $C_{max}$ was determined from

the actual measurements and AUC was determined by the trapezoidal method. These values were used in the following equation to calculate the bioavailability (BA(iv)):

$$\texttt{BA(iv)(\%) = (AUC/Dose)/(AUC(iv)/Dose(iv))}$$

where
AUC = AUC (ng•min/mL) after intestinal, intranasal or pulmonary administration;
Dose = dose ($\mu$g/kg) administered through intestinal, intranasal or pulmonary route;
AUC (iv) = AUC (ng•min/mL) after intravenous administration; and
Dose (iv) = dose ($\mu$g/kg) administered through intravenous route.

The results are shown in Table 8.

Table 8: Pharmacokinetic parameters after intravenous, intestinal, intranasal or pulmonary administration of hPTH(1-34) to rats.

[Table 8]

| Administration route | Dose | | $C_{max}$ | AUC | BA(iv) |
|---|---|---|---|---|---|
| | ($\mu$g/rat) | ($\mu$g/kg) | (ng/mL) | (ng·min/mL) | (%) |
| Intravenous administration | - | 10 | 31.00 ±1.30 | 208.6 ±52.7 | - |
| Intestinal administration | 100 | 378.0 ±36.8 | 0.93 ±0.80 | 32.3 ±30.2 | 0.4 ±0.4 |
| Intranasal administration | 200 | 822.2 ±19.2 | 0.67 ±0.16 | 43.9 ±11.6 | 0.3 ±0.1 |
| Pulmonary administration | 150 | 515.7 ±67.0 | 27.84 ±15.90 | 1534.1 ±1103.0 | 14.6 ±10.7 |

As can be seen from the results shown in Table 8, the hPTH(1-34) solution administered through the intravenous route in a dose of 10 $\mu$g/kg gave an AUC(iv) of 208.6 ng·min/mL. The hPTH(1-34) solution administered through the intestinal route in a dose of 100 $\mu$g/rat gave an AUC of 32.3 ng•min/mL. The hPTH(1-34) solution administered through the nasal route in a dose of 200 $\mu$g/rat gave an AUC of 43.9 ng•min/mL. The hPTH(1-34) solution administered through the pulmonary route in a dose of 150 $\mu$g/rat gave an AUC of 1534.1 ng· min/mL.

Thus, the BA(iv) values of hPTH(1-34) administered through intestinal, nasal or pulmonary route were determined to be 0.4%, 0.3% and 14.6%, respectively.

Based on the results of Comparative Example 9, the maximum plasma concentration ($C_{max}$) and the area under the plasma concentration-time curve (AUC) were calculated as the pharmacokinetic parameters from the change in the plasma concentration of hPTH(1-34) over time, as determined in the above-described tests of Examples 3 to 5, Example 8 and Comparative Example 9. These values were used to calculate the bioavailability (BA(iv)). The results are shown in Tables 9 to 12.

Table 9: Pharmacokinetic parameters after intestinal administration of hPTH(1-34) to rats (Enhanced absorption by C-CPE).

[Table 9]

| C-CPE | Dose | | $C_{max}$ (ng/mL) | AUC (ng·min/mL) | BA(iv) (%) |
|---|---|---|---|---|---|
| | ($\mu$g/rat) | ($\mu$g/kg) | | | |
| - | 100 | 378.0 ± 36.8 | 0.93 ± 0.80 | 32.3 ± 30.2 | 0.4 ± 0.4 |
| + | 100 | 403.8 ± 57.5 | 6.04 ± 4.34 | 243.6 ± 229.0 | 2.7 ± 2.1 |

As can be seen from the results shown in Table 9, the BA(iv) value of hPTH(1-34) was 2.7% with the intestinal administration of C-CPE 4 hours before the administration of hPTH(1-34). This means that, surprisingly, the BA(iv) of hPTH(1-34) preceded by administration of C-CPE was 6.3 times higher than the BA(iv) for hPTH(1-34) alone (0.4%), showing a marked increase.

Table 10: Pharmacokinetic parameters after intranasal administration of hPTH(1-34) to rats (Enhanced absorption by C-CPE).

[Table 10]

| C-CPE | Dose | | $C_{max}$ (ng/mL) | AUC (ng·min/mL) | BA(iv) (%) |
|---|---|---|---|---|---|
| | (μg/rat) | (μg/kg) | | | |
| - | 200 | 822.2 ± 19.2 | 0.67 ± 0.16 | 43.9 ± 11.6 | 0.3 ± 0.1 |
| + | 200 | 833.3 ± 0.0 | 3.58 ± 2.30 | 244.5 ± 109.5 | 1.4 ± 0.6 |

As can be seen from the results shown in Table 10, the BA(iv) value of hPTH(1-34) with the intranasal administration of C-CPE 4 hours before the administration of hPTH(1-34) was 1.4%, which was 4.6 times higher than the BA (iv) for hPTH(1-34) alone. This indicates that the pre-administration of C-CPE also markedly increased the BA(iv) of hPTH(1-34) administered through the nasal route.

Table 11: Pharmacokinetic parameters after pulmonary administration of hPTH(1-34) to rats (Enhanced absorption by C-CPE).

[Table 11]

| C-CPE | Dose | | $C_{max}$ (ng/mL) | AUC (ng·min/mL) | BA(iv) (%) |
|---|---|---|---|---|---|
| | (μg/rat) | (μg/kg) | | | |
| - | 150 | 515.7 ± 67.0 | 27.84 ± 15.90 | 1534.1 ± 1103.0 | 14.6 ± 10.7 |
| + | 150 | 498.7 ± 50.3 | 67.63 ± 17.87 | 3631.8 ± 1816.9 | 34.5 ± 16.3 |

As can be seen from the results shown in Table 11, the BA(iv) value of hPTH(1-34) with the pulmonary administration of C-CPE 4 hours before the administration of hPTH(1-34) was 34.5%, which was 2.4 times higher than the BA (iv) for hPTH(1-34) alone. This indicates that the pre-administration of C-CPE also markedly increased the BA(iv) of hPTH(1-34) administered through the pulmonary route.

Table 12: Pharmacokinetic parameters after pulmonary administration of hPTH(1-34) to rats (Enhanced absorption by CPE03 and CPE04).

[Table 12]

| C-CPE | Dose | | $C_{max}$ (ng/mL) | AUC (ng·min/mL) | BA(iv) (%) |
|---|---|---|---|---|---|
| | (μg/rat) | (μg/kg) | | | |
| - | 150 | 515.7 ± 67.0 | 27.84 ± 15.90 | 1534.1 ± 1103.0 | 14.6 ± 10.7 |
| CPE03 | 150 | 597.5 ± 33.0 | 96.02 ± 76.38 | 4040.9 ± 2190.3 | 33.0 ± 18.7 |
| CPE04 | 150 | 608.1 ± 49.2 | 134.77 ± 91.39 | 3631.8 ± 1816.9 | 35.6 ± 23.5 |

As can be seen from the results shown in Table 12, the BA(iv) values of hPTH(1-34) with the pulmonary administration of CPE03 and CPE04 2 hours before the administration of hPTH(1-34) were 33.0% and 35.6%, respectively, which were 2.3 times and 2.4 times higher than the BA(iv) for hPTH(1-34) alone, respectively. This indicates that the pre-administration of the C-CPE mutants also markedly increased the BA(iv) of hPTH(1-34) administered through the pulmonary route.

The results of Tables 9 to 12 indicate that the C-CPE or its mutants administered 2 to 4 hours before the administration of hPTH(1-34) markedly increased both the BA and the BA (iv) of hPTH(1-34) with respect to hPTH(1-34) administered alone, thus providing the evidence that the administration of the C-CPE or its mutants enhances the mucosal absorption of hPTH(1-34). This suggests that the mucosal absorption-enhancing agent and the composition for mucosal administration of the present invention can increase the bioavailability of peptide drugs administered through any of the intestinal, pulmonary and nasal mucosal routes to a level comparable to the highest bioavailability achieved by intravenous injection, thereby extending the administration route of peptide drugs that was otherwise limited to injections to the mucosal route.

The above-described examples demonstrate that the administration of the C-CPE or its mutants to serve as the mucosal absorption-enhancing agent enhance the absorption of peptide drugs, such as human parathyroid hormone hPTH(1-34), human ghrelin and human motilin, via the intestinal epithelial mucosa, nasal epithelial mucosa, respiratory tract epithelial mucosa or alveolar epithelial mucosa. Despite the fact that these examples are each an *in situ* experiment in which peptide drugs follow *in vivo* kinetics; they are susceptible to digestion by various proteases in the body, the absorption of the peptide drugs was markedly increased.

INDUSTRIAL APPLICABILITY

According to the present invention, there is provided a substance for use in a method for enhancing mucosal absorption of a peptide drug that enables oral, intranasal or pulmonary administration of peptide drugs whose administration route has heretofore been limited to the injections due to their poor absorption from the mucosa.
Specifically, the mucosal absorption of peptide drugs via intestinal, pulmonary or nasal route can be successfully enhanced by allowing the peptide drugs and the C-terminal fragment (C-CPE) of an enterotoxin (CPE) produced by the bacterium Clostridium perfringens of the genus Clostridium, in particular with C-CPE or mutants of C-CPE resulting from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE to act thereon. It has also been demonstrated that the absorption of the peptide drugs can be improved by allowing the C-CPE or its mutant to act prior to the administration of the peptide drugs. Since the composition for mucosal administration containing the C-CPE or its mutant can be administered via oral, intranasal and pulmonary administration routes that are less stressful to patients, it should find a wide range of industrial applications.

## Claims

1. A substance having an amino acid sequence of a C-terminal fragment (C-CPE) of an enterotoxin (CPE) produced by the bacterium *Clostridium perfringens* of the genus Clostridium for use in a method for enhancing mucosal absorption of a peptide drug.

2. The substance for use according to claim 1, wherein the substance having the amino acid sequence of the C-terminal fragment (C-CPE) of the enterotoxin (CPE) is C-CPE or a mutant resulting from deletion of one or several amino acid residues from amino acid residues 1 to 21 of the N-terminal of the C-CPE; most preferably a mutant resulting from deletion of amino acid residues 1 to 10 of the N-terminal of the C-CPE or a mutant resulting from deletion of amino acid residues 1 to 21 of the N-terminal of C-CPE.

3. The substance for use according to claim 1 or 2, wherein an amino acid sequence of the C-CPE is a sequence represented by SEQ ID NO: 1.

4. The substance for use according to claim 3, wherein a base sequence of the C-CPE is a sequence represented by SEQ ID NO: 2.

5. The substance for use according to claim 1, wherein an amino acid sequence of the mutant of the C-CPE is a sequence represented by SEQ ID NO: 3 or SEQ ID NO: 4.

6. A composition for mucosal absorption of a peptide drug, containing a peptide drug, preferably a peptide hormone, and the mucosal absorption-enhancing agent according to claim 1.

7. The composition for mucosal absorption of a peptide drug according to claim 6, wherein the peptide hormone is any of parathyroid hormone (PTH) and a derivative thereof, glucagon-like peptide-1, ghrelin, atrial natriuretic peptide, brain natriuretic peptide (BNP), C-type natriuretic peptide, insulin, motilin, leptin, resistin, glucagon, relaxin, galanin, gastrin, apelin, selectin, calcitonin, adrenomedullin, amylin, humanin, thymosin, endorphin, endomorphin, nocistatin, enkephalin, neuropeptide Y, neuropeptide S, neuromedin U, angiotensin, endothelin, guanylin, salusin, urotensin, oxytocin, vasopressin, neurophysin, melanocyte-stimulating hormone, urocortin, lipotropin, luteinizing hormone-releasing hormone, mystatin, prolactin-releasing peptide, somatostatin, cortistatin, thyrotropin-releasing hormone, substance P, neurokinin, endokinin, neurotensin, neoromedin N, obestatin, orexin, insulin-like growth factor-1 (IGF-1), melanin-concentrating hormone, corticotropin-releasing hormone, exendin-4, catacalcin, cholecystokinin, corticotrophin, melanotrophin, neoromedin C, copeptin, pituitary adenylate cyclase-activating peptide (PACAP), peptide YY, thyroliberin and a derivative thereof.

8. The composition for mucosal absorption of a peptide drug according to claim 6, wherein the peptide hormone is human parathyroid hormone or a derivative thereof (hPTH (1-34)), human ghrelin or human motilin.

9. The composition according to claim 6 for use in a method for enhancing mucosal absorption of a peptide drug, wherein the mucosal absorption is via intestinal epithelial mucosa, nasal epithelial mucosa, respiratory tract epithelial mucosa or alveolar epithelial mucosa.

10. The composition for mucosal absorption of a peptide drug according to claim 6, provided in a form of a powder preparation, an aqueous suspension or an oil suspension.

11. The mucosal absorption-enhancing agent according to claim 1 or 2 for use in a method for enhancing biological absorption of a peptide drug through mucosa.

12. The mucosal absorption-enhancing agent according to claim 1 or 2 for use according to claim 11, wherein the mucosal absorption-enhancing agent and the peptide drug are co-administered or are separately administered at an interval.

13. The mucosal absorption-enhancing agent according to claim 1 or 2 for use according to claim 11, wherein the mucosal absorption-enhancing agent is administered before the peptide drug is administered, preferably at least two hours, more preferably at least four hours before the peptide drug is administered.

14. The mucosal absorption-enhancing agent according to claim 1 to 2 for use according to claim 11, wherein the mucosal absorption occurs via intestinal epithelial mucosa, nasal epithelial mucosa, respiratory tract epithelial mucosa or alveolar epithelial mucosa.

15. The mucosal absorption-enhancing agent according to claim 1 or 2 for use according to claim 11, wherein the peptide drug is human parathyroid hormone or a derivative thereof (hPTH (1-34)), human ghrelin or human motilin.


**Patentansprüche**

1. Substanz, die eine Aminosäuresequenz eines C-terminalen Fragments (C-CPE) eines Enterotoxins (CPE), produziert durch das Bakterium *Clostridium perfringens* der Gattung Clostridium, hat, zur Verwendung in einem Verfahren zur Erhöhung der mukosalen Absorption eines Peptid-Arzneimittels.

2. Substanz zur Verwendung gemäß Anspruch 1, wobei die Substanz, die die Aminosäuresequenz des C-terminalen Fragments (C-CPE) des Enterotoxins (CPE) hat, C-CPE oder eine Mutante, resultierend aus einer Deletion eines oder mehrerer Aminosäurereste von Aminosäureresten 1 bis 21 des N-Terminus des C-CPE; am bevorzugtesten eine Mutante, resultierend aus einer Deletion von Aminosäureresten 1 bis 10 des N-Terminus des C-CPE, oder eine Mutante, resultierend aus einer Deletion von Aminosäureresten 1 bis 21 des N-Terminus von C-CPE, ist.

3. Substanz zur Verwendung gemäß Anspruch 1 oder 2, wobei eine Aminosäuresequenz des C-CPE eine Sequenz, dargestellt durch SEQ ID NO:1, ist.

4. Substanz zur Verwendung gemäß Anspruch 3, wobei eine Basensequenz des C-CPE eine Sequenz, dargestellt durch SEQ ID NO:2, ist.

5. Substanz zur Verwendung gemäß Anspruch 1, wobei eine Aminosäuresequenz der Mutante des C-CPE eine Sequenz, dargestellt durch SEQ ID NO:3 oder SEQ ID NO:4, ist.

6. Zusammensetzung zur mukosalen Absorption eines Peptid-Arzneimittels, die ein Peptid-Arzneimittel, vorzugsweise ein Peptidhormon, und das die mukosale Absorption verstärkende Mittel gemäß Anspruch 1 umfasst.

7. Zusammensetzung zur mukosalen Absorption eines Peptid-Arzneimittels gemäß Anspruch 6, wobei das Peptidhormon ein beliebiges von Nebenschilddrüsenhormon (PTH) und einem Derivat davon, Glucagon-artigem Peptid-1, Ghrelin, atrialem nutriuretischem Peptid, nutriuretischem Gehirnpeptid (BNP), nutriuretischem Peptid C-Typ, Insulin, Motilin, Leptin, Resistin, Glucagon, Relaxin, Galanin, Gastrin, Apelin, Selectin, Calcitonin, Adrenomedullin, Amylin, Humanin, Thymosin, Endorphin, Endomorphin, Nocistatin, Enkephalin, Neuropeptid Y, Neuropeptid S, Neuromedin U, Angiotensin, Endothelin, Guanylin, Salusin, Urotensin, Oxytocin, Vasopressin, Neurophysin, Melanocyten-stimulierendem Hormon, Urocortin, Lipotropin, luteinisierendes Hormon-freisetzendem Hormon, Mystatin, Prolactin-freisetzendem Peptid, Somatostatin, Cortistatin, Thyrotropin-freisetzendem Hormon, Substanz P, Neurokinin, Endokinin, Neurotensin, Neoromedin N, Obestatin, Orexin, insulinartigem Wachstumsfaktor-1 (IGF-1), Melaninkonzentrierendem Hormon, Corticotropin-freisetzendem Hormon, Exendin-4, Catacalcin, Cholecystokinin, Corticotrophin, Melanotrophin, Neoromedin C, Copeptin, Hypophysenadenylatcyclaseaktivierendem Peptid (PACAP), Peptid YY, Thyroliberin und einem Derivat davon ist.

8. Zusammensetzung zur mukosalen Absorption eines Peptid-Arzneimittels gemäß Anspruch 6, wobei das Peptidhormon humanes Nebenschilddrüsenhormon oder ein Derivat davon (hPTH (1-34)), humanes Ghrelin oder humanes Motilin ist.

9. Zusammensetzung gemäß Anspruch 6 zur Verwendung in einem Verfahren zur Verstärkung der mukosalen Absorption eines Peptid-Arzneimittels, wobei die mukosale Absorption über die intestinale Epithelmucosa, die nasale Epithelmucosa, die Epithelmucosa des respiratorischen Trakts oder die alveoläre Epithelmucosa erfolgt.

10. Zusammensetzung zur mukosalen Absorption eines Peptid-Arzneimittels gemäß Anspruch 6, das in Form eines Pulverpräparats, einer wässrigen Suspension oder einer Ölsuspension bereitgestellt ist.

11. Die mukosale Absorption verstärkendes Mittel gemäß Anspruch 1 oder 2 zur Verwendung in einem Verfahren zur Verstärkung der biologischen Absorption eines Peptid-Arzneimittels durch Mucosa.

12. Die mukosale Absorption verstärkendes Mittel gemäß Anspruch 1 oder 2 zur Verwendung gemäß Anspruch 11, wobei das die mukosale Absorption verstärkende Mittel und das Peptid-Arzneimittel co-verabreicht werden oder getrennt mit einem Intervall verabreicht werden.

13. Die mukosale Absorption verstärkendes Mittel gemäß Anspruch 1 oder 2 zur Verwendung gemäß Anspruch 11, wobei das die mukosale Absorption verstärkende Mittel verabreicht wird, bevor das Peptid-Arzneimittel verabreicht wird, vorzugsweise wenigstens zwei Stunden, bevorzugter wenigstens vier Stunden bevor das Peptid-Arzneimittel verabreicht wird.

14. Die mukosale Absorption verstärkendes Mittel gemäß Anspruch 1 oder 2 zur Verwendung gemäß Anspruch 11, wobei die mukosale Absorption durch intestinale Epithelmucosa, nasale Epithelmucosa, Epithelmucosa des respiratorischen Trakts oder alveoläre Epithelmucosa erfolgt.

15. Die mukosale Absorption verstärkendes Mittel gemäß Anspruch 1 oder 2 zur Verwendung gemäß Anspruch 11, wobei das Peptid-Arzneimittel ein humanes Nebenschilddrüsenhormon oder ein Derivat davon (hPTH (1-34)), humanes Ghrelin oder humanes Motilin ist.


**Revendications**

1. Substance ayant une séquence d'acides aminés d'un fragment C-terminal (C-CPE) d'une entérotoxine (CPE) produite par la bactérie *Clostridium perfringens* du genre Clostridium pour une utilisation dans un procédé d'amplification de l'absorption mucosale d'un médicament peptidique.

2. Substance pour une utilisation selon la revendication 1, où la substance ayant la séquence d'acides aminés du fragment C-terminal (C-CPE) de l'entérotoxine (CPE) est le C-CPE ou un mutant résultant de la délétion d'un ou plusieurs résidus d'acides aminés à partir des résidus d'acides aminés 1 à 21 de l'extrémité N-terminale du C-CPE ; de manière préférée entre toutes, un mutant résultant de la délétion des résidus d'acides aminés 1 à 10 de l'extrémité N-terminale du C-CPE ou un mutant résultant de la délétion des résidus d'acides aminés 1 à 21 de l'extrémité N-terminale du C-CPE.

3. Substance pour une utilisation selon la revendication 1 ou 2, où une séquence d'acides aminés du C-CPE est une séquence représentée par SEQ ID NO : 1.

**4.** Substance pour une utilisation selon la revendication 3, où une séquence de bases du C-CPE est une séquence représentée par SEQ ID NO : 2.

**5.** Substance pour une utilisation selon la revendication 1, où une séquence d'acides aminés du mutant du C-CPE est une séquence représentée par SEQ ID NO : 3 ou SEQ ID NO : 4.

**6.** Composition pour une absorption mucosale d'un médicament peptidique, contenant un médicament peptidique, de préférence une hormone peptidique, et l'agent amplifiant l'absorption mucosale selon la revendication 1.

**7.** Composition pour une absorption mucosale d'un médicament peptidique selon la revendication 6, où l'hormone peptidique est l'une quelconque de l'hormone parathyroïdienne (PTH) et un dérivé de celle-ci, le peptide 1 de type glucagon, la ghréline, le peptide natriurétique atrial, le peptide natriurétique cérébral (BNP), le peptide natriurétique de type C, l'insuline, la motiline, la leptine, la résistine, le glucagon, la relaxine, la galanine, la gastrine, l'apeline, la sélectine, la calcitonine, l'adrénomédulline, l'amyline, l'humanine, la thymosine, l'endorphine, l'endomorphine, la nocistatine, l'encéphaline, le neuropeptide Y, le neuropeptide S, la neuromédine U, l'angiotensine, l'endothéline, la guanyline, la salusine, l'urotensine, l'oxytocine, la vasopressine, la neurophysine, l'hormone stimulant les mélanocytes, l'urocortine, la lipotropine, l'hormone de libération de l'hormone lutéinisante, la mystatine, le peptide de libération de la prolactine, la somatostatine, la cortistatine, l'hormone de libération de la thyrotropine, la substance P, la neurokinine, l'endokinine, la neurotensine, la neuromédine N, l'obestatine, l'orexine, le facteur de croissance de type insulinique 1 (IGF-1), l'hormone de concentration de la mélanine, l'hormone de libération de la corticotropine, l'exendine 4, la catacalcine, la cholécystokinine, la corticotrophine, la mélanotrophine, la neuromédine C, la copeptine, le peptide activateur de l'adénylate cyclase hypophysaire (PACAP), le peptide YY, la thyrolibérine et un dérivé de ceux-ci.

**8.** Composition pour une absorption mucosale d'un médicament peptidique selon la revendication 6, où l'hormone peptidique est l'hormone parathyroïdienne humaine ou un dérivé de celle-ci (hPTH (1-34)), la ghréline humaine ou la motiline humaine.

**9.** Composition selon la revendication 6 pour une utilisation dans un procédé d'amplification de l'absorption mucosale d'un médicament peptidique, où l'absorption mucosale se produit par l'intermédiaire de la muqueuse de l'épithélium intestinal, la muqueuse de l'épithélium nasal, la muqueuse de l'épithélium des voies respiratoires ou la muqueuse de l'épithélium alvéolaire.

**10.** Composition pour une absorption mucosale d'un médicament peptidique selon la revendication 6, fournie sous la forme d'une préparation pulvérulente, d'une suspension aqueuse ou d'une suspension huileuse.

**11.** Agent amplifiant l'absorption mucosale selon la revendication 1 ou 2 pour une utilisation dans un procédé d'amplification de l'absorption biologique d'un médicament peptidique à travers une muqueuse.

**12.** Agent amplifiant l'absorption mucosale selon la revendication 1 ou 2 pour une utilisation selon la revendication 11, où l'agent amplifiant l'absorption mucosale et le médicament peptidique sont coadministrés ou sont administrés séparément à un certain intervalle.

**13.** Agent amplifiant l'absorption mucosale selon la revendication 1 ou 2 pour une utilisation selon la revendication 11, où l'agent amplifiant l'absorption mucosale est administré avant l'administration du médicament peptidique, de préférence au moins deux heures, de manière davantage préférée au moins quatre heures avant l'administration du médicament peptidique.

**14.** Agent amplifiant l'absorption mucosale selon la revendication 1 ou 2 pour une utilisation selon la revendication 11, où l'absorption mucosale se produit par l'intermédiaire de la muqueuse de l'épithélium intestinal, la muqueuse de l'épithélium nasal, la muqueuse de l'épithélium des voies respiratoires ou la muqueuse de l'épithélium alvéolaire.

**15.** Agent amplifiant l'absorption mucosale selon la revendication 1 ou 2 pour une utilisation selon la revendication 11, où le médicament peptidique est l'hormone parathyroïdienne humaine ou un dérivé de celle-ci (hPTH (1-34)), la ghréline humaine ou la motiline humaine.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5695956 A, McClane, Bruce A
- US 2006084594 A1, Santin, Alessandro D
- WO 2006047214 A2
- JP 6107557 A
- EP 0474453 A
- EP 08790688 A
- JP 2007171676 A

### Non-patent literature cited in the description

- **WONG V. ; GUMBINER BM.** *Journal of Cell Biology,* 1997, vol. 136, 399-409
- **TAVELIN S. et al.** *Molecular Pharmacology,* 2003, vol. 64, 1530-1540
- **KONDOH M. et al.** *Yakugaku Zasshi,* 2007, vol. 127 (4), 601-609
- *Journal of Cell Biology,* 1999, vol. 147 (1), 195-204
- **KONDOH M. et al.** *Molecular Pharmacology,* 2005, vol. 67, 749-756
- **VAN ITALLIE CM. et al.** *Journal of Biological Chemistry,* vol. 283, 268-274
- **MASUYAMA A. et al.** *Journal of Pharmacology and Experimental Therapeutics,* 2005, vol. 314, 789-95
- **EBIHARA C. et al.** *Biochemical Pharmacology,* 2007, vol. 73, 824-30
- **HARADA M. et al.** *Biochemical Pharmacology,* 2007, vol. 73, 206-14
- **TAKAHASHI A. et al.** *Biochemical Pharmacology,* 2008, vol. 75, 1639-48
- *J. Cell Biol.,* vol. 136, 1239-47